(19) **Europäisches Patentamt / European Patent Office / Office européen des brevets**

(11) **EP 2 803 729 B1**

(12) **EUROPÄISCHE PATENTSCHRIFT**

(45) Veröffentlichungstag und Bekanntmachung des Hinweises auf die Patenterteilung:
**04.07.2018 Patentblatt 2018/27**

(51) Int Cl.:
*C12P 5/02* (2006.01)        *C12M 1/107* (2006.01)
*C12M 1/16* (2006.01)        *C12M 1/00* (2006.01)
*C12M 1/04* (2006.01)        *C12M 1/33* (2006.01)
*C12M 1/06* (2006.01)

(21) Anmeldenummer: **14168299.7**

(22) Anmeldetag: **14.05.2014**

(54) **Verfahren zur Herstellung von Biogas durch kontinuierliche Vergärung von Substratmischungen und Biogasanlage zur kontinuierlichen Vergärung von Substratmischungen**

Process for the production of biogas by continuous fermentation of substrate mixtures and biogas plant for the continuous fermentation of substrate mixtures

Procédé pour la production de biogaz avec fermentation en continu de mélanges de substrat et installation de biogaz destinée à la fermentation en continu de mélanges de substrat

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2013 DE 102013104966**

(43) Veröffentlichungstag der Anmeldung:
**19.11.2014 Patentblatt 2014/47**

(73) Patentinhaber: **Agraferm GmbH**
**85276 Pfaffenhofen (DE)**

(72) Erfinder:
• **Rossberg, Andreas**
 **39443 Staßfurt (DE)**
• **Harders, Dirk**
 **24107 Kiel (DE)**
• **Sternekieker, Mirko**
 **15345 Altlandsberg (DE)**
• **Lehmann, Tommy**
 **37355 Gerterode (DE)**
• **Kube, Jürgen**
 **85276 Pfaffenhofen (DE)**
• **Mutke, Bernd**
 **56729 Weiler (DE)**

(74) Vertreter: **Patronus IP Patent- und Rechtsanwälte Neumarkter Strasse 18**
**81673 München (DE)**

(56) Entgegenhaltungen:
EP-A1- 1 795 585        DE-A1-102006 035 794
DE-A1-102009 009 985

• KUSCH ET AL: "Biogas production with horse dung in solid-phase digestion systems", BIORESOURCE TECHNOLOGY, ELSEVIER BV, GB, Bd. 99, Nr. 5, 15. Dezember 2007 (2007-12-15), Seiten 1280-1292, XP022391678, ISSN: 0960-8524, DOI: 10.1016/J.BIORTECH.2007.02.008

**Beschreibung**

Die Erfindung betrifft ein Verfahren zur Herstellung von Biogas durch kontinuierliche einstufige Vergärung von Substratmischungen sowie eine Biogasanlage zur kontinuierlichen Vergärung von Substratmischungen. Insbesondere betrifft die Erfindung ein Verfahren zum Betreiben einer Biogasanlage zur kontinuierlichen Vergärung einer Substratmischung, wobei die Substratmischung überwiegend Tiermist, insbesondere Pferdemist, enthält.

Tiermist bzw. Pferdemist wird als Substrat in bestehende Biogasanlagen in kleinen Mengen zugeführt. Bei den meisten Biogasanlagen besteht die Substratmischung überwiegend aus nachwachsenden pflanzlichen Rohstoffen. Wird der Anteil von Tiermist im Substrat erhöht, dann verursacht dies in Biogasanlagen zahlreiche Probleme.

Einerseits hat sich gezeigt, dass mit der Betriebsdauer die Viskosität im Fermenter ansteigt. Dies erfordert eine Herabsetzung des Trockenrückstandes (TR), denn eine hohe Viskosität benötigt viel Energie zum Betätigen des Rührwerkes im Fermenter bzw. kann die Drehbewegung des Rührwerkes des Fermenters vollständig zum Stillstand bringen.

Tiermist, vor allem Pferdemist, enthält oftmals einen hohen Anteil an Sand, der in Behältern und Rohrleitungen zu einem erhöhten Verschleiß der Pump- und Rühreinrichtungen führt.

Tiermist, vor allem Pferdemist, kann ein sehr heterogenes Substrat sein, da Pferde oftmals auf Stroh oder Sägespäne stehen. Dabei kann das Stroh zum Teil ungehäckselt sein (Langstroh) und Faserlängen von mehr als 50 cm aufweisen. Solche Pferdemist-Chargen haben einen TR von über 40% und eine sehr geringe Schüttdichte. Andere Pferdemist-Chargen bestehen fast nur aus Pferdekot und weisen einen geringen TR von etwa 20-30% und eine hohe Schüttdichte auf. Stapelt man diese Chargen, so kompaktiert das Substrat zu einer klebrigen Masse, welche die Misch- und Förderschnecken verstopft.

Pferdemist ist meistens stapelfähig. Lagert man Pferdemist zu lange, setzt ein Kompostierungsprozess ein. Kompostierter Pferdemist weist einen geringeren Glühverlust (GV) und einen höheren TR als frischer Pferdemist auf. Durch die Kompostierung sinkt auch die oTR-spezifische Biogasausbeute des Pferdemists.

Die DE 10 2010 019 321 A1 beschreibt ein Verfahren zur energetischen Verwertung von Pferdemist. Dabei wird der Pferdemist mittels Siebung in einen festen und flüssigen Bestandteil separiert und der flüssige Bestandteil vergoren. Die Abwärme der Biogasnutzung dient zur Trocknung des festen Bestandteils des Pferdemists. Die Bezeichnung flüssiger Pferdemist dient in diesem Zusammenhang als Bezeichnung für den Siebdurchgang. Gesiebter Pferdemist ist fest bis pastös.

In der EP 1 301 583 B1 und in der DE 10 2010 024 639 A1 ist ein Verfahren beschrieben, bei dem Pferdemist mit einer sogenannten Trockenfermentation vergoren wird. Als Trockenfermentation wird hierbei die diskontinuierliche, absatzweise Fermentation von stapelfähigen Substraten in Garagen-ähnlichen Fermentern bezeichnet. Der Pferdemist wird dabei in den Garagen gestapelt, die Garagen werden verschlossen und das Substrat dann mit Perkolat besprüht. Nach einer Gärdauer von einigen Wochen wird die Garage geöffnet und der vergorene Pferdemist entnommen. Für dieses Verfahren ist es wichtig, dass der ausgegorene Pferdemist noch stapelfähig ist, da ansonsten die Drainageleitungen der Garagen verstopfen. Besonders strohhaitiger Pferdemist ist für dieses Verfahren geeignet.

Das Sammeln verschiedener Pferdemistchargen und der Transport zur Biogasanlage kann in einem Container durchgeführt werden, der in der DE 10 2004 029 854 A1 beschrieben wird.

Saskia Oldenburg (Doktorandin an der TU Hamburg-Harburg) et al. werben im Internet (http://www.sciencestarter.de/pfen) (abgerufen am 25.04.2013) für ein Forschungsprojekt zur Entwicklung eines Aufbereitungskonzeptes für Pferdemist, welches die (Co-)Vergärung in einer konventionellen Biogasanlage ermöglicht. Es sollen die Grundlagen für die Nutzung von Pferdemist in Biogasanlagen gelegt werden, indem ein theoretisches Konzept in die Praxis umgesetzt wird. Mit dem Bau einer Pilotanlage im Technikumsmaßstab sollen Versuchsdaten gewonnen werden. Der Pferdemist soll in einer Pilotanlage aufbereitet werden, indem Störstoffe, Sand und Stroh abgetrennt und die Pferdeäpfel in Wasser gelöst werden. So soll das Stroh extern aufbereitet und die gelösten Pferdeäpfel mit einem konstanten Wassergehalt direkt der Biogasanlage zugegeben werden können.

Matthias Mönch-Tegeder beschreibt im Internet (https://www.uni-hohenheim.de/news/strom-aus-pferdeaepfeln-agrartechniker-machen-pferdemistbiogasfaehig-4) (abgerufen am 25.04.2013) ein Projekt Universität Hohenheim mit dem Titel "Strom aus Pferdeäpfeln: Agrartechniker machen Pferdemist biogasfähig": Demnach findet Pferdemist bisher kaum Verwendung, da er sich nur bedingt als Dünger eignet und deshalb oft kostenpflichtig entsorgt werden muss. Nur in sehr kleinen Mengen werde er bereits zur Energiegewinnung eingesetzt. Das Problem am Pferdemist sei die große Menge Stroh darin, die bewirkt, dass der Mist in der Biogasanlage auf der übrigen Biomasse schwimmt. Dieses Problem wird mit einer Vorbehandlung mittels eines Querstromzerspaner gelöst. Durch die Vorbehandlung wird die Oberfläche des Pferdemistes vergrößert und er verbindet sich gut mit dem übrigen Gärsubstrat im Fermenter.

Ein Hydrolysereaktor, der zur Vergärung von überwiegend Tiermist eingesetzt wird, ist in der DE 10 2008 015 609 A1 beschrieben. Der Einsatz einer Hydrolysevorstufe bei der Vergärung von Pferdemist ist bekannt. Die Hydrolsevorstufe ist ein der Biogas-Produktion vorgeschalteter Behälter mit einem deutlich geringeren pH-Wert und sehr kurzer Aufenthaltszeit von ca. zwei Tagen. Dort findet eine teilweise Verflüssigung des Substrates (Hydrolyse) und Produktion von organischen Säuren (Acidogenese) statt. Ein Großteil des Substrates wird erst im nachfolgenden Biogasreaktor hydrolysiert und umgesetzt. Biogasanlagen mit Hydrolyse weisen eine erhöhte Stabilität im Vergleich zu einstufigen Biogasanlagen gegenüber Substratwechseln auf. Ein systemischer Nachteil der Hydrolyse ist, dass sie am besten in stark verdünnten Systemen arbeitet, da bei einer Akkumulation von ca. 20 g/L organischen Säuren die A-cidogenese zum Erliegen kommt. Feuchte Substrate sind für Hydrolysereaktoren daher besser geeignet als trockene Substrate. Ein weiterer Nachteil ist die Produktion von energiereichen Hydrolysegasen (Wasserstoff, $H_2S$, $CO_2$ und geringe Mengen Methan bei der Verwendung von Anmaischströmen), die oftmals ungenutzt in die Umgebung entweichen und für Geruchsbelästigung sorgen.

In EuroHeat&Power, "Trennung von Hydrolyse und Methanisierung, Vom wertlosen Pferdemist zur wertvollen Energie", 40. Jg. (2011), Heft 3, Seiten 38 ff wird eine solche Biogasanlage zur Vergärung von Pferdemist beschrieben. Diese Biogasanlage ist zweistufig aufgebaut, d.h. eine erste Stufe für eine Hydolyse und eine zweite Stufe für eine Methanisierung. Die Trennung von Hydrolyse und Methanisierung sei bei einer Strohvergärung unumgänglich. Stroh mit einer Schutzschicht aus Wachs und einem hohen Zelluloseanteil sei nur im Sauren Milieu bei einem pH-Wert von unter sieben zerlegbar und zu vergären. In einer einstufigen Biogasanlage bei einem pH-Wert von sieben bis acht im Fermenter durchschwimme das Stroh den Fermenter. Dies kann nur durch eine aufwendige Aufbereitung mit hohen Kosten vermieden werden.

Häufig werden Aggregate zur mechanischen Zerkleinerung auf Biogasanlagen eingesetzt, die Pferdemist nur in geringen Mengen einsetzen. Diese Aggregate können Hammermühlen (DE 20 2008 006 843 U1), Prallreaktoren (DE 10 2005 021 503 A1), Querstromzerspanner (DE 10 2007 037 187 A1) oder Extruder (DE 10 2009 058 588 A1, DE 10 2010 010 091 A1) sein. Auch thermisch-chemische Verfahren sind bekannt, z.B. das Organosolv-Verfahren (DE 10 2010 006 609 A1 und WO 2010 043 424 A1), die gezielt den Strohanteil des Pferdemists aufschließen.

Der Nachteil der mechanischen und thermischen Vorbehandlungen ist der hohe Energiebedarf und der mechanische Verschleiß. So gibt ein Hersteller von Extrudern zur Biomassezerkleinerung einen Energiebedarf von 75 bis 95 kWh/t für den Aufschluss von Stroh mit 85% TR an. Da eine Biogasanlage ca. 500 kWh elektrische Energie aus einer Tonne Stroh erzeugen kann, stellt der Energiebedarf der Aufbereitung bereits 17% der produzierten Leistung dar. Konventionelle Biogasanlagen für landwirtschaftliche Substrate haben normalerweise einen Eigenenergiebedarf von 5% bis 8% für die gesamte Anlage. Der Energiebedarf einer Zerkleinerung kann mit der Formel von Bond abgeschätzt werden.

$$W = 100 \cdot \alpha \cdot \left( \frac{1}{x_{80,2}^{0,5}} - \frac{1}{x_{80,1}^{0,5}} \right)$$

Mit

$\alpha$      [kWh $mm^{0,5}$/t] Bond-Parameter (Work-Index)
$X_{80,2}$      [m] Größe Produkt
$X_{80,1}$      [m] Größe Edukt

Der Term $x_{80}$ bedeutet, dass 80% der Partikelmasse eine Größe kleiner als $x_{80}$ hat. Für Stroh gilt im cm-Maßstab ein Bond-Index von ca. 1,4 kWh $mm^{0,5}$/t. Er steigt, wenn feiner zerkleinert wird. Zerkleinert man Stroh von 25 cm auf 10 cm, ergibt sich ein Energiebedarf von ca. 5 kWh/t. Bei einer Zerkleinerung auf 1 cm werden 35 kWh/t benötigt, bei einer Zerkleinerung auf 0,1 cm sind hingegen 130 kWh/t notwendig. Die empirische Formel ist nicht ganz exakt, zeigt aber die Tendenz auf.

Auch sind nicht alle Vorbehandlungsmethoden gegen Störstoffe resistent. Eine energetische Verwertungsmethode, die auch ohne Vorbehandlung betrieben werden kann, wäre daher wünschenswert.

Ein zum laminaren Durchmischen eines Fermenterinhaltes geeignetes Rührwerk ist in der EP 2 064 308 B1 beschrieben.

Bei landwirtschaftlichen Biogasanlagen wird der TR üblicherweise auf ca. 8% in den Fermentern eingestellt. Dies ist zum Beispiel im Biogasmessprogramm I (BMEVL, FNR: Ergebnisse des Biogasmessprogramms, Gülzow, 2005) in Kapitel 8.3.1 (Substratcharakteristik - Gehalt an TR und oTR) dokumentiert. Die meisten Anlagen werden dort im Bereich von 4% bis 8% TR gerührt; nur eine Anlage weist einen TR von knapp über 10% auf. Auch in dem nachfolgenden Biogasmessprogramm II (BMEVL, FNR: Biogasmessprogramm II - 61 Biogasanlagen im Vergleich, Gülzow, 2010) wurde der TR im Fermenter in Kapitel 6.2.2.1 dokumentiert. Hier liegen die Werte im Ablauf des Fermenters im Mittel um 5%, maximal bei 12%.

Zum Trennen der Bestandteile des vergorenen Gärsubstrates sind Pressschneckenseparatoren aus der DE 42 32 449 B4 und Walzenseparatoren aus der DE 39 41 916 A1 bekannt.

Große Störstoffe können mit einem Macerator zerkleinert oder abgetrennt werden. Ein solcher Macerator geht aus dem deutschen Gebrauchsmuster DE 20 2006 003 816 U1 hervor.

Die EP 2 215 241 B1 beschreibt ein Verfahren zur Herstellung von Biogas mit einem Biogasreaktor, wobei das Substrat mehr als 30 Gew.-% Geflügelkot enthält. Dem Biogasreaktor wird ein Substratgemisch aus einem ersten Substrat (Geflügelkot) und einem zweiten Substrat (Biogene Reststoffe und/oder nachwachsende pflanzliche Rohstoffe wie Mais oder dergleichen) zugeführt. Dem Substrat wird Frischwasser und/oder Prozesswasser zugeführt. Hierdurch ergibt sich eine pumpfähige Masse mit einem Gehalt an Trockensubstanz (TS) im Bereich von 10 - 25 Gew.-% Vorzugsweise weist diese Masse einen Gehalt an Trockensubstanz von 15 -25 Gew.-% auf. Das vergorene Gärsubstrat wird einer Flüssig-/Festphasentrennvorrichtung zugeführt, wie z.B. einem Dekanter. Einem Dekanter kann kein Gärsubstrat mit einem hohen Gehalt an Trockensubstanz zugeführt werden. Aus A. Rushton et al., "Solid-Liquid Filtration and Separation Technology", WILEY-VCH Verlag GmbH, 2000, Kapitel 8.2.5.1 (Seite 310) geht hervor, dass einem Dekanter vergorenes Gärsubstrat ("digested sludge") mit einem Feststoffanteil von 2 bis 4 Gew.-% zugeführt werden kann. Daher begrenzt der Dekanter den maximalen Gehalt an Trockensubstanz im Biogasreaktor.

Aus der DE 10 2005 061 039 A1 geht ein langgestreckter Fermenter hervor, in dem sich am Boden ein Rohr befindet, das einen Strömungskanal begrenzt. Ein erstes Rührwerk wird mittels eines Tauchmotors angetrieben und erzeugt eine Strömung durch den Strömungskanal hindurch. Oberhalb des Strömungskanals bildet sich eine Strömung, die entgegen zur Strömungsrichtung im Strömungskanal gerichtet ist und an ihrer Oberseite eine Schwimmschicht mitnimmt. Ein zweites Rührwerk mit einem Tauchmotor ist zur Ausbildung einer turbulenten Strömung vorgesehen, um die Schwimmschicht am Ende des Behälters aufzulösen. Im Bereich der Schwimmschichtauflösung wird vermehrt Biogas freigesetzt.

In der DE 10 2006 035 794 A1 ist eine Biogasanlage zum Vergären von Inkontinenzprodukten (IKP) beschrieben. Die Inkontinenzprodukte betreffen vor allem benutzte Windeln. Diese enthalten üblicherweise Cellulosefasern, Kunststofffolien und Fäkalien. Diese Abfälle werden zunächst mechanisch aufbereitet. In einem Bioreaktor können diese Abfälle vergoren werden. Das nach der Methanisierung anliegende Faulgut wird einem Trennbehälter zugeführt und dort in eine kunststoffreiche Fraktion, eine wässrige Zone und eine faserstoffreiche Fraktion aufgetrennt. Die faserstoffreiche Fraktion werden einer Entwässerungseinrichtung und die kunststoffreiche Fraktion einer Entwässerungspresse zugeführt. Prozesswasser wird von einer zentralen Dosierstation einem Pulper zugeführt. Eine Zweigleitung ist vorgesehen, mit der Prozesswasser auch dem Trennbehälter zum Trennen der kunststoffreichen Fraktion und der faserstoffreichen Fraktion zugeführt bzw. abgezogen werden kann.

Der Erfindung liegt die Aufgabe zu Grunde, zumindest einen Nachteil der oben erläuterten Verfahren zum Betreiben einer Biogasanlage zu überwinden.

Eine weitere Aufgabe der Erfindung liegt in der Schaffung eines Verfahrens zum Betreiben einer Biogasanlage, mit welcher Tiermist enthaltender Fermenterinhalt mit hoher Effizienz vergärt werden kann.

Eine weitere Aufgabe der Erfindung liegt in der Schaffung einer Biogasanlage, mit welcher eine Feinstoffe, wie z.B. Sand, enthaltende Substratmischung zuverlässig und dauerhaft vergärt werden kann.

Die unabhängigen Ansprüche lösen eine oder mehrere der oben angegebenen Aufgaben. Vorteilhafte Ausgestaltungen der Erfindung sind in den jeweiligen Unteransprüchen angegeben.

Gemäß einem ersten Aspekt betrifft die Erfindung ein Verfahren zur Herstellung von Biogas durch kontinuierliche einstufige Vergärung von Substratmischungen mit einem Biogasfermenter, einer Gärsubstratseparationseinheit zum Trennen des im Biogasfermenter vergorenen Gärsubstrates in Gärrest und Prozesswasser, wobei Prozesswasser teilweise in den Biogasfermenter zurück geführt wird. Dieses Verfahren zeichnet sich dadurch aus, dass die Substratmischung mindestens 50 Gew% Tiermist enthält und dass die Menge an zurück geführtem Prozesswasser derart eingestellt wird, dass im Biogasfermenter der Fermenterinhalt einen Trockenrückstand (TR) von zumindest 16% aufweist.

Eine einstufige Vergärung bedeutet, dass im Fermenter sowohl Hydrolyse und Methansierung stattfinden und keine zwei getrennten Fermenter für die Hydrolyse bzw. Methanisierung vorgesehen sind.

Bei der einstufigen Vergärung beträgt der pH-Wert mindestens 6,5. Bei einem herkömmlichen Fermenter für eine Hydrolyse ist der pH-Wert hingegen kleiner als 6,5.

Der Fermenter ist vorzugsweise als ein Rührkessel bestehend aus einem Fermenterbehälter und einem oder mehreren Rührwerken ausgebildet, d.h. ist weitgehend frei von Konzentrations- bzw. Temperaturgradienten.

Eine Substratmischung, welche überwiegend Tiermist enthält, ist eine Substratmischung, welche zumindest 50 Gew.% Tiermist enthält. Tiermist umfasst tierische Ausscheidungen, auch mit Einstreu, insbesondere Stroh, Sägemehl oder anderes pflanzliches Material, das im Rahmen der Tierhaltung zugefügt worden ist, oder mit Futterresten vermischt, dessen TR 15% übersteigt.

Wie es oben erläutert ist, wird bei landwirtschaftlichen Produkten der TR üblicherweise im Bereich von ca. 4% bis 8% in Fermentern von Biogasanlagen eingestellt. Ein höherer TR wäre wünschenswert, da hierdurch die Effizienz erheblich gesteigert werden kann. Ein höherer TR bedeutet, dass sich im Fermenter weniger Wasser und mehr in Biogas umsetzbare Anteile an Substrat bei gleicher Fermentergröße befinden. Mit zunehmendem TR nimmt jedoch die Viskosität zu, so dass mehr Energie aufgewendet werden muss, um den Fermenterinhalt zu rühren oder die Bewegung des Fermenterinhaltes sogar unmöglich werden kann. Daher hat sich für den Dauerbetrieb eine Obergrenze von ca. 8% TR für landwirtschaftliche Gärsubstrate durchgesetzt.

Überraschenderweise hat sich herausgestellt, dass sich bei einer Substratmischung, die überwiegend Tiermist, insbesondere Pferdemist enthält, eine wesentlich geringere Viskosität als bei einer überwiegend auf nachwachsenden Rohstoffen basierenden Substratmischung einstellt. Die Viskosität errechnet sich nach dem Fließgesetzt von Ostwald und deWaele gemäß

$$\eta = k \cdot \dot{y}^{m-1}$$

Mit

$\eta$   [Pas] dynamische Viskosität
$k$   [Pas$^m$] Konsistenzfaktor
$\dot{y}$   [s$^{-1}$] Schergeschwindigkeit
m   [-] Fließindex

Es wird angenommen, dass in Tiermist Wasser physikalisch an Feinstoffe gebunden ist und einen Matsch bildet. Feinstoffe im Sinne der vorliegenden Erfindung sind kleine Fasern, insbesondere Fasern mit einer Länge von bis 1 mm. Dieser Matsch "schmiert" den Fermenterinhalt, so dass selbst wenn der Fermenterinhalt die Viskosität erhöhende Stoffe, wie z.B. lange Fasern enthält, die Viskosität dennoch so gering ist, dass mit einem TR von zumindest 16% die Biogasanlage auf Dauer wirtschaftlich betrieben werden kann und der energetische Aufwand zum Rühren des Fermenterinhaltes gering ist.

Der Anteil an Tiermist in der Substratmischung beträgt vorzugsweise zumindest 75 Gew.%, 80 Gew.%, 90 Gew.% und insbesondere zumindest 95 Gew.%.

Wenn Pferdemist mit einem TR von 8% vergärt werden würde, dann müssten der Fermenter sehr groß sein, um einen entsprechend stark verdünnten Pferdemist in ausreichender Menge vergären zu können. Mit der Erfindung wird aufgrund des hohen Trockenrückstands ein hoher Durchsatz bei relativ geringem Fermentervolumen erzielt.

Da bei der Erfindung der Trockenrückstand zumindest 16 % aufweist, ist der Wassergehalt gering und dadurch ist die Neigung gering, dass im Fermenter die Sandpartikel sedimentieren. Die Sandpartikel bleiben zumindest mit dem überwiegenden Anteil in Schwebe und werden mit dem Gärrest aus dem Fermenter abgeführt.

Mit dem erfindungsgemäßen Verfahren ist es sogar möglich einen Fermenterinhalt mit einer Flüssigphase zu vergären, die bei einer Temperatur von 40°C und einer Scherrate von 10 s$^{-1}$ eine Viskosität von mindestens 200 mPas, insbesondere mindestens 300 mPas oder mindestens 400 mPas oder sogar mindestens 500 mPas aufweist. Solch hohe Viskositäten in der Flüssigphase werden durch die im Tiermist, insbesondere im Pferdemist, enthaltenen Feinteile verursacht. Diese Feinteile bewirken auch einen hohen Trockenrückstand in der Flüssigphase, der bei diesen Substratmischungen mindestens 7% oder mindestens 9% und insbesondere mindestens 11% beträgt.

Die Erfinder haben festgestellt, dass es entgegen den Konzepten im oben erläuterten Stand der Technik es nicht notwendig ist, Pferdemist enthaltende Substratmischungen vorab von Störstoffe, wie Sand und Stroh, zu trennen oder die Substratmischungen mittels einer separaten Hydrolyse aufzubereiten und erst dann einer Methanisierung zu unterziehen. Vielmehr können viele Probleme der Vergärung von Tiermist dadurch beseitigt werden, indem ein hoher Trockenrückstand im Biogasfermenter eingestellt wird.

Bei einem solch hohen TR-Gehalt bleibt das Stroh im Fermenterinhalt gleichmäßig verteilt. In einem herkömmlichen Fermenter ist der Fermenterinhalt dünnflüssiger, wodurch das Stroh aufschwimmt und durch den Fermenter hindurchgeleitet wird. Da bei dem hohen TR-Gehalt das Stroh gleichmäßig im Fermenterinhalt verteil ist, wird es abgebaut und in Biogas umgesetzt.

Bei der Erfindung ist es nicht notwendig, das Prozesswasser aufwändig zu reinigen und die Feststoffe fast vollständig zu entfernen. Der TR-Gehalt des zurück geführten Prozesswassers kann zumindest 1%, zumindest 2%, zumindest 3% und insbesondere zumindest 6% betragen. Der TR-Gehalt des zurück geführten Prozesswassers sollte möglichst nicht größer als 11% sein.

Das Prozesswasser wird vorzugsweise direkt in den Biogasreaktor zurück geführt. Ein Zuführen von Prozesswasser alleine durch Anmaischen des Pferdemists macht es schwierig im Biogasreaktor den gewünschten hohen TR-Anteil zu erzielen.

Nach einem zweiten Aspekt der vorliegenden Erfindung wird ein Verfahren zur Herstellung von Biogas durch kontinuierliche einstufige Vergärung eines Fermenterinhalts mit einem Biogasfermenter vorgesehen, bei dem eine Feinstoffe enthaltende Substratmischung vergärt wird, die einen TR-Gehalt von zumindest 16% aufweist, und der Fermenterinhalt mit einem Hauptrührwerk, bestehend aus zwei Rührwerken oder mehr, laminar umgewälzt wird, und im Bereich benachbart zur oberen Oberfläche des Fermenterinhaltes dieses mit einem Nebenrührwerk turbulent umgewälzt wird.

Die Erfinder der vorliegenden Erfindung haben festgestellt, dass Feinstoffe enthaltende Substratmischungen Anteile an Biogas im Fermenterinhalt zurück behalten und der Fermenterinhalt ähnlich wie ein Hefeteig aufgehen kann. Es wird angenommen, dass kleine Bläschen aus Biogas an den Feinstoffen hängen bleiben und die Feinstoffe als Blasenbildungskeime dienen und das Koaleszieren der Biogasblasen verhindern. Dieses Problem tritt vor allem bei einer Substratmischung mit einem TR-Gehalt von etwa 10% auf und nimmt mit zunehmenden TR-Gehalt zu. Mit dem erfindungsgemäßen Verfahren können auch Substratmischungen mit einem höheren TR von zumindest 16% vergoren werden.

Das zum laminaren Umwälzen vorgesehene Hauptrührwerk ist vorzugsweise ein Rührwerk mit vertikal angeordneter Rührwelle, so dass der Fermenterinhalt laminar, kreisförmig und etwa horizontal umgewälzt wird.

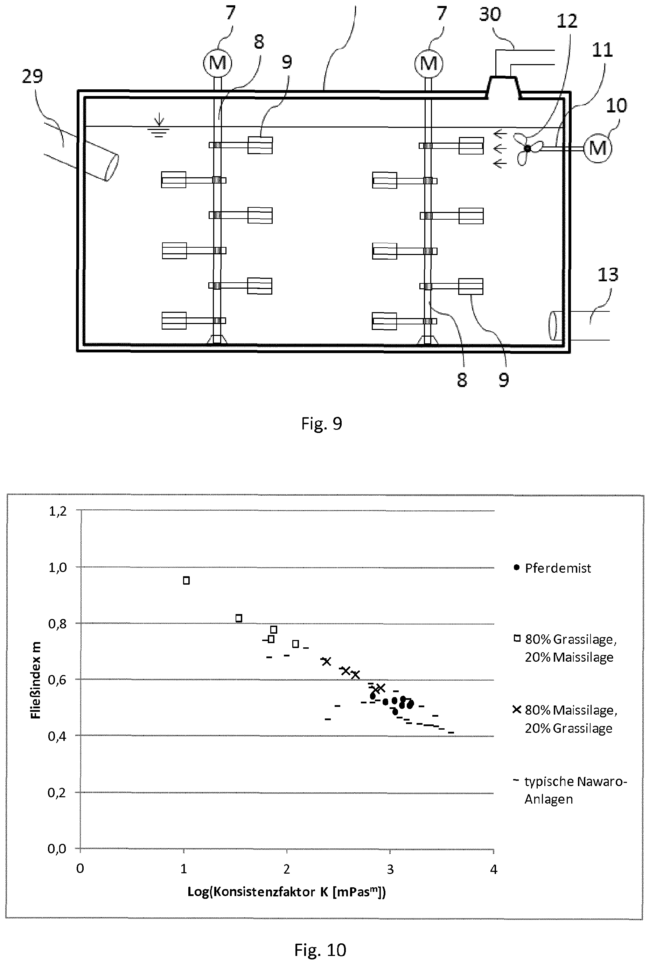

Die Feinstoffe treten auch in konventionellen Biogasanlagen auf, insbesondere bei Anlagen die viel Grassilage vergären. Eine weitere Eigenschaft des Fermenterinhalts von Biogasanlagen nach der vorliegenden Erfindung ist die hohe Viskosität des Prozesswassers, wie in Figur 10 zu sehen ist. Zwar haben auch andere Biogasanlagen eine hohe Viskosität im Prozesswasser, insbesondere hochbelastete Biogasanlagen, die viel Getreide oder Getreide-Ganzpflanzensilage vergären. Bei allen Referenzanlagen, deren Proben in Figur 10 zu sehen sind, hat das Prozesswasser einen TR von <11%. Das Problem der Gasblasenakkumulation tritt jedoch nicht auf. Die Erfinder vermuten daher, dass die Kombination des hohen Anteils der Blasenbildungskeime und der hohen Viskosität für das schlechte Entgasungsverhalten verantwortlich ist. Eine solche Kombination tritt vor allem in Biogasanlagen auf, die überwiegend Tiermist vergären.

Die Blasenauftriebsgeschwindigkeit kann mit der Formel von Stokes in erster Näherung beschrieben werden. Genaugenommen gilt die Formel nur für Newtonsche Fluide und kugelförmige Blasen. Ähnliche Zusammenhänge gelten aber auch im Biogasreaktor.

$$v_g = \frac{d^2 \cdot g \cdot (\rho_l - \rho_g)}{18 \cdot \eta}$$

Dabei ist

$v_g$     Blasenaufstiegsgeschwindigkeit
$g$     Erdbeschleunigung
$\rho_l$     Dichte der Flüssigphase
$\rho_g$     Dichte der Gasphase
$\eta$     Viskosität der Flüssigphase

Es zeigt sich, dass die Blasenaufstiegsgeschwindigkeit mit steigender Viskosität und fallendem Blasendurchmesser sinkt. Der Gas-Volumenanteil $\varepsilon$ (oder auch Gas-Holdup, bzw. Porosität) des Fermenterinhaltes wird beschrieben über

$$\varepsilon = \frac{\dfrac{\dot{V}_g}{v_g}}{\dfrac{\dot{V}_g}{v_g} + \dfrac{\dot{V}_l}{v_l}}$$

Dabei ist

$V_l$     mittlere Abströmgeschwindigkeit der Flüssigphase
$\dot{V}_g$     Gas-Volumenstrom
$\dot{V}_l$     Volumenstrom der Flüssigphase

Wenn die Blasenaufstiegsgeschwindigkeit sehr klein wird, nähert sich der Gas-Volumenanteil dem Wert 1, d.h. der Fermenterinhalt besteht nur noch aus Gasphase. Im Biogasreaktor wird normalerweise ein $\varepsilon$ von 1% bis 10% erreicht. Die Verweilzeit der Flüssigphase liegt bei 20 bis 80 Tagen, während die Gasproduktion ca. 1 bis 10 m$^3$ Gas pro m$^3$ Reaktor und Tag beträgt. Der Gas-Volumenstrom ist also deutlich größer als der Flüssigkeitsvolumenstrom.

Im Falle von vielen kleinen Blasen in hochviskoser Umgebung kann es also zu einem Ansteigen des Gas-Volumenanteils des Fermenterinhaltes kommen.

Es wurde festgestellt, dass das "Aufgehen" des Fermenterinhaltes durch Zugabe von Antischaum-Mittel oder Pflanzenöl (z.B. mit einer Menge von 0,3 L/m$^3$) verhindert werden kann, was die Hypothese der Bindung von kleinen Bläschen an den Feinstoffen stützt.

Da beim erfindungsgemäßen Verfahren im Bereich benachbart zur oberen Oberfläche des Fermenterinhaltes diese mit einem Nebenrührwerk turbulent umgewälzt wird, werden lokal starke Scherraten erzielt, die das Biogas von den Feinstoffen lösen und so das Biogas sicher und zuverlässig freisetzen. Hierdurch wird die Zugabe von teuren Anti-Schaummitteln oder Pflanzenöl vermieden und die Biogasanlage kann selbst mit einer Feinstoffe enthaltenden Substratmischung zuverlässig betrieben werden.

Vorzugsweise wird im Wesentlichen der gesamte Fermenterinhalt laminar umgewälzt und lediglich in einem kleinen Bereich wird die laminare Strömung von der turbulenten Strömung überlagert.

Das Hauptrührwerk weist vorzugsweise eine kleinere Reynoldszahl als das Nebenrührwerk auf.

Das Hauptrührwerk kann mit einer Reynoldszahl von nicht mehr als 50 und das Nebenrührwerk mit einer Reynoldszahl von zumindest 100 betrieben werden.

Gemäß einem dritten Aspekt der vorliegenden Erfindung ist eine Biogasanlage zur kontinuierlichen Vergärung von überwiegend Tiermist enthaltenden Substratmischungen, insbesondere Pferdemist enthaltenden Substratmischungen, mit einem einstufigen Biogasfermenter, einer Separationseinheit zum Trennen des im Biogasfermenter vergorenen Gärsubstrates in Gärrest und Prozesswasser vorgesehen.

Diese Biogasanlage zeichnet sich dadurch aus, dass die Separationseinheit einen Vorlagehomogenisierungstank aufweist, wobei von einem Prozesswassertank eine Rezirkulatleitung zum Vorlagehomogenisierungstank führt, um Prozesswasser zum Einstellen des Trockenrückstandes vor dem Separieren in die Separationseinheit zurückzuführen.

Die Separationseinheit weist vorzugsweise einen Sandabscheider auf.

Der einstufige Biogasfermenter ist vorzugsweise zum Vergären von einer Substratmischung mit einem Trockenrückstand von zumindest 16% ausgebildet. Hierbei beträgt der organische Trockenrückstand vorzugsweise zumindest 8%, insbesondere zumindest 9% oder zumindest 10%.

Die oben beschrieben unterschiedlichen Aspekte können auch in Kombination angewandt werden.

Alle Prozentangaben (%) sind Gewichtsprozentangaben, sofern nichts anderes eindeutig definiert ist.

Die Erfindung wird nachfolgend anhand der Zeichnungen näher erläutert. Die Zeichnungen zeigen in:

Figur 1      eine Tabelle zur Massenbilanz eines Biogasreaktors, in dem eine Substratmischung mit überwiegend Tiermist mit unterschiedlichem Trockenrückstand vergärt wird,

Figur 2      eine Tabelle der Trockenrückstände bei der Siebung von Prozesswasser aus einer Gärsubstratseparation mittels eines Schwingsiebs mit unterschiedlichen Spaltsieben,

Figur 3      den Füllstand in einem Fermenter, in dem Feinstoffe enthaltender Fermenterinhalt vergärt wird, wobei der Füllstand mit einer RadarFüllstandsmessung und mit einer hydrostatischen Füllstandsmessung gemessen wird und der Fermenterinhalt ausschließlich laminar umgewälzt wird,

Figur 4      der Füllstand in einem Fermenter, in dem Feinstoffe enthaltender Fermenterinhalt vergärt wird, wobei der Füllstand mit einer RadarFüllstandsmessung und mit einer hydrostatischen Füllstandsmessung gemessen wird und der Fermenterinhalt zumindest bereichsweise turbulent umgewälzt wird,

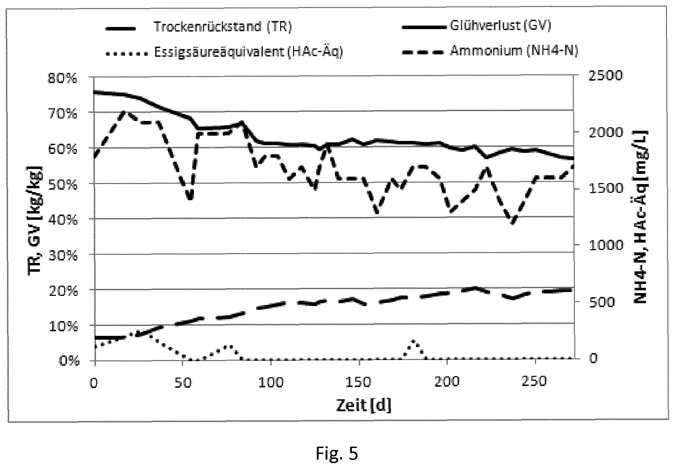

Figur 5      die Ganglinien beim Hochfahren eines Fermenters auf eine kontinuierliche Volllastvergärung bei ca. 20% TR, wobei die Fermenterbiologie stabile Prozessparameter aufzeigt,

Figur 6      ein Ablaufdiagramm eines Verfahrens zum Betreiben einer Biogasanlage zur kontinuierlichen Vergärung einer Substratmischung, wobei die Substratmischung überwiegend Tiermist enthält,

Figur 7      schematisch den Aufbau einer Biogasanlage als Blockschaltbild,

Figur 8      eine Biogasanlage zur Durchführung des erfindungsgemäßen Verfahrens in der Draufsicht,

Figur 9      schematisch vereinfacht einen Fermenter in einer Querschnittsdarstellung,

Figur 10     eine Auftragung des Fließindex von temperaturabhängigen Fließkurven bei Feinstoffe enthaltendem Prozesswasser gegen den Konsistenzfaktor unterschiedlicher Substratmischungen, und

Figur 11     die Viskosität des Prozesswassers unterschiedlicher Substratmischungen von einem mesophil betriebenen Fermenter in Abhängigkeit von der Scherrate.

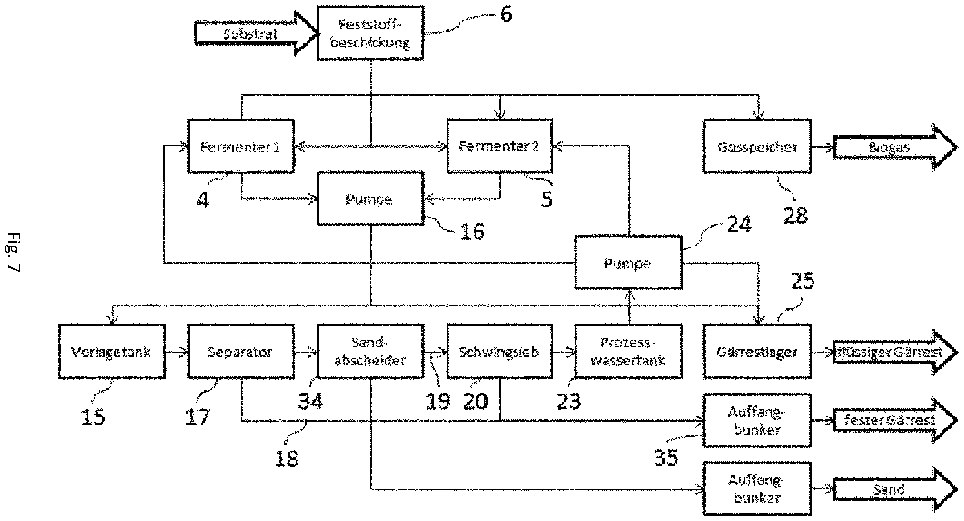

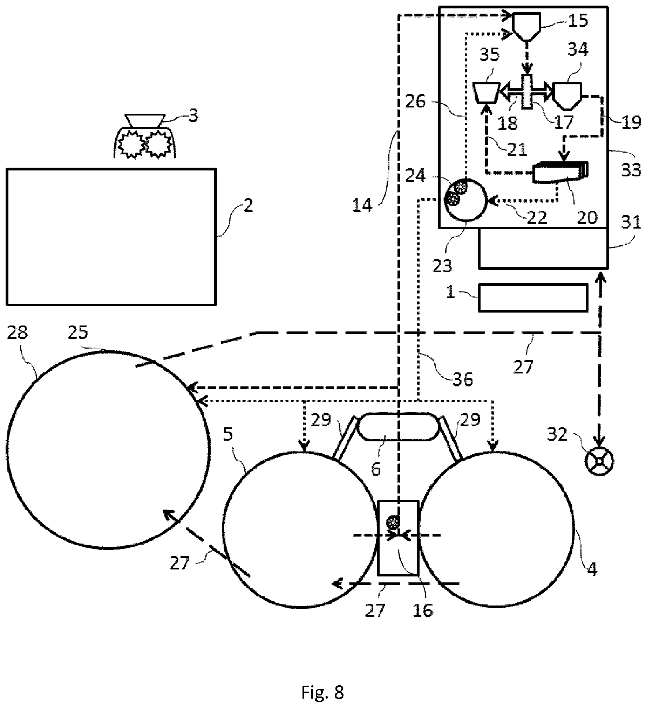

Ein Ausführungsbeispiel einer erfindungsgemäßen Biogasanlage (Figur 7, 8) weist eine Waage 1 zum Wiegen der angelieferten Substrate und eine Siloplatte 2 auf. Auf der Waage 1 werden die das Substrat anliefernden Lastwagen vor und nach dem Abliefern des Substrates gewogen, so dass die angelieferte Masse des Substrates feststellbar ist. Das Substrat wird auf der Siloplatte 2 abgeladen. Hier kann das Substrat optisch auf Eignung kontrolliert werden.

Benachbart zur Siloplatte 2 ist ein Schredder 3 vorgesehen, in dem bei Bedarf das Substrat gehäckselt werden kann. Die Häcksellänge nach der Aufbereitung beträgt typischerweise 5 cm bis 25 cm, insbesondere 5 cm bis 15 cm und vorzugsweise etwa 10 cm.

Die Biogasanlage weist einen ersten Fermenter 4 und einen zweiten Fermenter 5 auf. Die Fermenter sind einstufige Fermenter, d.h., dass in den Fermenter sowohl die Hydrolyse als auch die Methanisierung erfolgt. Der pH-Wert im Fermenter beträgt zumindest 6,5. Der pH-Wert kann auch zumindest 7 bzw. 7,5 betragen.

Zum Zuführen der Substratmischung in die beiden Fermenter 4, 5 ist eine Feststoffbeschickung 6 vorgesehen. Die Feststoffbeschickung weist einen Beschickungsbehälter auf, der an zwei Fördereinrichtungen 29 gekoppelt ist, so dass die Substratmischung automatisch aus dem Beschickungsbehälter der jeweiligen Fördereinrichtung 29 abgeführt und von dieser dem jeweiligen Fermenter 4, 5 zugeführt wird. Die Fördereinrichtungen 29 führen die Substratmischung am oberen Randbereich der jeweiligen Fermenter 4, 5 zu. Die Fördereinrichtungen 29 sind beispielsweise Förderbänder oder Förderschnecken.

Der Beschickungsbehälter der Feststoffbeschickung 6 kann als sogenannter Futtermischwagen ausgebildet sein, der mehrere vertikal stehende Schnecken enthält, mit welchen die Substrate durchmischt werden und zu einer zur Fördereinrichtung gerichteten Austragsöffnung befördert werden. Bei überwiegend Tiermist enthaltenden Substraten hat sich jedoch die Ausbildung des Beschickungsbehälters als Abschiebecontainer als vorteilhaft herausgestellt. Ein Abschiebecontainer weist eine etwa vertikal stehende und in horizontaler Richtung bewegliche Schubplatte auf, die den Inhalt des Containers in Richtung zu einer Austragsöffnung drückt, so dass der Inhalt des Abschiebecontainers zuverlässig der Fördereinrichtung zugeführt werden kann. Benachbart zur Austragsöffnung des Abschiebecontainers sind vorzugsweise eine oder mehrere Fräswalzen vorgesehen, die die Substratmischung auflockern und förderfähig machen. Die Fräswalzen des Abschiebecontainers können auch derart ausgebildet sein, dass sie zusätzlich die Funktion des Schredders ausführen, der dann weggelassen werden kann.

Die beiden Fermenter 4, 5 sind in der Draufsicht kreisförmig ausgebildet und haben einen Durchmesser im Bereich von 15 m bis 20 m und eine lichte Höhe von ca. 6 m bis 10 m. Die Fermenter 4, 5 sind Behälter mit einer Bodenplatte, Seitenwandung und Deckenplatte. Die Bodenplatte ist in der Regel eine vor Ort gegossene Betonplatte. Die Seitenwandungen und die Deckenplatte bestehen vorzugsweise aus Fertigbeton-Bauteilen. In der Deckenplatte kann eine gewichtsbelastete Überlaufklappe integriert sein, welche bei einer Fehlbedienung des Fermenters, bei der der darin befindliche Fermenterinhalt auf ein Volumen anschwillt, sich öffnet, so dass überschüssiger Fermenterinhalt austreten kann. Diese gewichtsbelastete Überlaufklappe bildet somit ein Sicherheitsventil, das bei einem Überdruck im Fermenter ein Bersten desselben verhindert.

Die Fermenter weisen jeweils als Hauptrührwerk mit zumindest zwei und vorzugsweise drei vertikale Paddelrührwerke 7 auf, wie sie in der EP 2 064 308 B1 beschrieben sind. Die Paddelrührwerke 7 haben jeweils einen Durchmesser von ca. 2,5 m bis 3,5 m. Sie werden jeweils von einem Motor mit einer maximalen Leistung von 10 kW angetrieben. Die Paddelrührwerke 7 werden langsam laufend mit einer Drehzahl von etwa 6 U/min bis 10 U/min betrieben. Hierdurch wird der gesamte Fermenterinhalt laminar im Fermenter umgewälzt.

Die Fermenter weisen jeweils als Nebenrührwerk einen Stabmixer 10 auf. Der Stabmixer 10 besitzt eine horizontale Welle 11 mit einem Propeller 12, der im Betrieb den Fermenterinhalt in eine Richtung parallel zur Welle 11 beaufschlagt. Der Stabmixer 10 wird von einem Motor angetrieben, der eine maximale Leistung von etwa 10 kW bis 15 kW besitzt. Mit dem Stabmixer wird ein Teil des Fermenterinhaltes turbulent umgewälzt. Hierdurch entstehen innerhalb des Fermenterinhalts Schubspannungen, welche das Biogas aus dem Fermenterinhalt lösen.

Die Rührwerke 7,10 sind vorzugsweise an eine Notstromversorgung angeschlossen, so dass bei Stromausfall sichergestellt ist, dass der Fermenterinhalt weiterhin umgewälzt wird und Biogas hieraus entweicht.

Die Fermenter 4, 5 weisen jeweils am unteren Bereich eine Austragsöffnung 13 auf, von welcher eine Leitung 14 zu einem Vorlagehomogenisierungstank 15 in einer Separationseinheit 33 führt. In der Leitung 14 ist eine Pumpe 16 angeordnet, mit welcher der Abzug des vergorenen Gärsubstrates aus dem jeweiligen Fermenter 4, 5 gesteuert werden kann. Die Pumpe 16 ist beispielsweise als Excenterschneckenpumpe mit einer Förderleistung von etwa 10 m³/h bis 20 m³/h ausgebildet.

Der Vorlagehomogenisierungstank 15 ist quaderförmig oder würfelförmig mit einem Volumen von ca. 0,5 m³ bis 5 m³ und insbesondere von ca. 0,5 m³ bis 2 m³ ausgebildet. Er weist einen Boden in der Form eines Pyramidenstumpfes auf. Das Volumen ist so zu bemessen, dass das Gärsubstrat nicht ruhig steht, die Verweilzeit kurz ist und nicht sedimentieren kann. Die Größe des Vorlagehomogenisierungstanks 15 ist somit in Abhängigkeit der Strömung des Gärsubstrates und der Konsistenz des Gärsubstrates einzustellen. Im vorliegenden Ausführungsbeispiel wird zumindest jede Stunde aus den Fermentern 4, 5 vergorenes Gärsubstrat abgezogen und der Separationseinheit zugeführt. Da das Gärsubstrat aus überwiegend Tiermist besteht, ist die Dichte der meisten Feststoffanteile im Gärsubstrat dem von Wasser ähnlich, so dass die Neigung des vergorenen Gärsubstrates sich zu entmischen gering ist. Dies gilt vor allem für die organischen Feststoffanteile, die den überwiegenden Teil der Feststoffanteile darstellen. Es ist nicht notwendig, einen Rührer im Vorlagehomogenisierungstank vorzusehen, um eine Sedimentierung zu vermeiden. Der Vorlagehomogenisierungstank 15 kann mit einem Füllstandsensor und einer Überfüllsicherung versehen sein.

Ein am Boden befindlicher Auslass des Vorlagehomogenisierungstankes 15 ist mit einer ersten Separationsstufe verbunden. Im vorliegenden Ausführungsbeispiel umfasst die erste Separationsstufe zwei Pressschneckenseparatoren 17 mit einer Siebkorblänge von etwa 0,5 m bis 1,5 m. Die Siebkörbe weisen eine Spaltenweite von 500 $\mu$m bis 1500 $\mu$m und vorzugsweise 800 $\mu$m bis zu 1200 $\mu$m und insbesondere etwa 1000 $\mu$m auf.

Mit den Pressschneckenseparatoren 17 wird der feste Gärrest vom Prozesswasser getrennt. Von den Pressschneckenseparatoren 17 führt eine Fördereinrichtung 18, beispielsweise in Form eines Förderbandes, zu einem Auffangbunker 35, in dem die festen Gärreste gelagert werden.

Die Separationseinheit 33 weist einen Sandabscheider 34 mit einer Grundfläche von etwa 1 m$^2$ und einem Volumen von etwa 1 m$^3$ auf. Der Überlauf des Sandabscheiders 34 wird mittels einer Leitung 19 zu einer zweiten Separationsstufe 20 geführt. Vorzugsweise ist der Sandabscheider 34 bodenseitig konisch bzw. pyramidenstumpfartig ausgeführt. Der Sandabscheider 34 weist an seiner tiefsten Stelle eine Öffnung zum Abziehen des Sediments auf, so dass dieses täglich abgezogen werden kann.

Vor der Separatoreinheit 17 kann ein Macerator mit Störstoffschleuse angeordnet sein. Ein solcher Macerator ist zum Beispiel in der DE 20 2006 003 816 U1 beschrieben. Der Macerator dient zum Trennen von großen Störteilen, wie zum Beispiel Hufeisen, aus dem Gärsubstrat. Er weist ein Lochblech mit darauf angeordneten, sich drehenden Messern auf, so dass große Störteile aus dem durch das Lochblech getriebenen, abgebauten Gärsubstrat entfernt werden können.

Der Überlauf bzw. Überstand der Abtrennvorrichtung wird mittels einer Fördereinrichtung 21 direkt oder über die Fördereinrichtung 18 zum Auffangbunker 35 befördert. Im vorliegenden Ausführungsbeispiel ist die zweite Separationsstufe 20 als Schwingsieb ausgebildet. Die zweite Separationsstufe 20 zum Abtrennen von Feststoffen kann auch als Dekanter oder einer ähnlich geeigneten Vorrichtung zum Abtrennen von Feststoffen ausgeführt sein.

Die erste und zweite Separationsstufe können auch durch einen Dekanter ersetzt werden. Die Separationseinheit 33 ist dann einstufig ausgebildet.

Das Filtrat der zweiten Separationsstufe 20 läuft über eine Leitung 22 in einen Prozesswassertank 23. Der Prozesswassertank 23 kann als Schacht unmittelbar unterhalb der zweiten Separationsstufe 20 ausgebildet sein, so dass das Filtrat der zweiten Separationsstufe ohne zusätzliche Leitung direkt aus der zweiten Separationsstufe in den Prozesswassertank 23 läuft. Der Prozesswassertank 23 ist mit einer Pumpeneinheit 24 verbunden. Die Pumpeneinheit 24 weist eine erste Pumpe und eine zweite Pumpe auf. Mit der ersten Pumpe wird Prozesswasser über eine Rezirkulatleitung 36 in die Fermenter 4, 5 und in ein Gärrestlager 25 gepumpt. Die erste Pumpe ist beispielsweise als Excenterschneckenpumpe mit einer Förderleistung von etwa 25 m$^3$/h bis 40 m$^3$/h ausgebildet. Die Förderleistung der ersten Pumpe ist derart einstellbar, dass sich in den Fermentern 4, 5 der gewünschte Trockenrückstand einstellt. Bei Bedarf können in den Zuleitungen zu den einzelnen Fermentern 4, 5 Drosselventile vorgesehen sein, sodass die Ströme an Prozesswasser zu den einzelnen Fermentern 4, 5 individuell einstellbar sind. Dies ist insbesondere zweckmäßig, wenn die beiden Fermenter 4, 5 unterschiedlich groß sein sollten.

Die zweite Pumpe dient zum Fördern von Prozesswasser über eine Rezirkulatleitung 26 zum Vorlagehomogenisierungstank 15. Die zweite Pumpe ist beispielsweise als Kreiselpumpe oder peristaltische Pumpe mit einer Förderleistung von 15 bis 25 m$^3$/h ausgebildet. Die Rezirkulatleitung 26 ist mit einem Drosselventil (nicht dargestellt) versehen, mit welchem die dem Vorlagehomogenisierungstank 15 zugeführte Menge an Prozesswasser gesteuert werden kann. Wenn eine überwiegend Pferdemist enthaltende Substratmischung vollständig ausgegoren wird, dann weist der Gärrest einen TR von bspw. knapp über 28% auf. Dann ist es zweckmäßig Frischwasser dem Vorlagehomogenisierungstank zuzuführen, damit ein derart trockener Gärrest in zwei Fraktionen getrennt werden kann.

Die erste Pumpe und die zweite Pumpe sind unabhängig voneinander ansteuerbar.

Die Fermenter 4, 5 sind mittels Gasleitungen 27 mit einem Gasspeicher 28 verbunden. Die Gasleitungen 27 sind an einer Auslassöffnung 30 in der Deckenwandung des jeweiligen Fermenters angeschlossen. Der Gasspeicher 28 und das Gärrestlager 25 können gemeinsam aus einem oder mehreren Behältern ausgebildet sein, wobei sich in einem jeden Behälter der flüssige Gärrest und darüber das erzeugte Biogas befinden. Ein solcher Gasspeicher ist beispielsweise in der EP 2 535 402 A1 beschrieben. Der Gasspeicher 28 ist vorzugsweise als Doppelmembrangasspeicher ausgebildet, welcher eine innere Membran und eine äußere Membran aufweist, wobei ein Gebläse zum Einstellen eines Tragluftdruckes zwischen den beiden Membranen vorgesehen ist. Die äußere Membran ist in Betrieb etwa kugelförmig aufgespannt. Die Höhe der inneren Membran wird über die Aufnahme und Abgabe des Biogases mittels des Tragluftdruckes derart gesteuert, dass sich die jeweiligen temperaturkorrigierten Füllstande der Gasspeicher entsprechen.

Der Biogasspeicher 28 kann auch mehrere Behälter umfassen, wobei diese dann vorzugsweise so gekoppelt sind, dass deren Gasspeicherfüllstände auf gleichem Niveau gehalten werden, indem der Tragluftdruck nach Maßgabe eines mit Hilfe eines Füllstandsensors gemessenen Füllstandes gesteuert wird.

Bei dem in Figur 7 gezeigten Ausführungsbeispiel wird das Gärsubstrat von der Pumpe 16 aus den Fermentern 4, 5 direkt in den Vorlagehomogenisierungstank 15 und von dort zur Separationseinheit 33 gefördert. In einer alternativen Ausgestaltung können die Fermenter 4, 5 mit einer Leitung zum Gärrestlager 25 verbunden sein, so dass abgebautes Gärsubstrat aus den Fermentern 4, 5 zunächst zum Gärrestlager 25 befördert wird. Hier kann noch eine Nachvergärung stattfinden, insbesondere wenn das Gärrestlager 25 zusammen mit dem Gasspeicher 28 als kombinierter Gärrestlager/Gasspeicher ausgebildet ist. Bei einer solchen Ausführungsform ist vom Gärrestlager 25 zum Vorlagehomogenisierungstank 15 eine Leitung vorzusehen, mit welcher die Gärreste aus dem Gärrestlager 25 dem Vorlagehomogenisierungstank 15 zugeführt werden können. Diese Ausführungsform hat auch den Vorteil, dass bei einer Unterbrechung des Betriebs der Separationseinheit 33 abgebaute Gärsubstrate kontinuierlich aus den Fermentern 4, 5 abgezogen und im Gärrestlager 25 zwischengespeichert werden können. Die Fermenter können dann weiter mit der Substratmischung und Prozesswasser beschickt werden.

Das Biogas kann aus dem Gasspeicher einem Verbraucher, wie z.B. einem Blockheizkraftwerk 31 zugeführt werden, oder in ein Erdgas- bzw. Biogasnetz eingespeist werden. Für den Fall, dass gebildetes Biogas weder einem Gasnetz noch einem Verbraucher zugeführt werden kann, ist eine Notfackel 32 zum kontrollierten Abbrennen des Biogases installiert.

Der flüssige Gärrest kann dem Gärrestlager 25 entnommen und als Dünger verwendet werden, wobei er in flüssiger Form auf Feldern ausgetragen wird.

Nachfolgend wird der Betrieb der oben beschriebenen Biogasanlage erläutert. Beim nachfolgend erläuterten Ausführungsbeispiel wird eine Substratmischung von 80% Pferdemist und 20% Energiepflanzensilage verwendet. Im Rahmen der Erfindung kann auch anstelle von Pferdemist anderer Tiermist in der Substratmischung enthalten sein. Tiermist enthält etwa 15 % bis 80 % Trockenmasse, die in der Regel Einstreu, Fasern, Sand und Feinstoffe (≤1 mm) umfassen. Bei Tiermist, insbesondere Pferdemist, ist ein Großteil der Feinstoffe in Wasser suspendierbar. Nach dem Zentrifugieren von Fermenterinhalt der oben beschriebenen Biogasanlage, wurde ein Überstand mit einem TR von 6 bis 7 Gew.% festgestellt.

Besteht eine Substratmischung hingegen überwiegend aus nachwachsenden Rohstoffen, dann weist das entstehende Gärsubstrat auch Fasern und Feinstoffe, die zum Teil gelöst sind, auf. Dabei ist jedoch entweder die Menge an Feinstoffen wesentlich geringer oder die Viskosität der Flüssigphase niedriger, was an einem geringeren TR des Prozesswassers erkennbar ist.

Die Flüssigphase des Fermenterinhalts ist definiert als das Filtrat einer Siebung des Fermenterinhalts mit einer Plansiebmaschine mit einer Maschenweite von 500 $\mu$m. Solche Plansiebmaschinen erlauben ein Sieben mit kreisender Siebbewegung gemäß DIN 53 477.

Die in der Flüssigphase gelösten Feinstoffe sind schlecht entwässerbar, da Wasser an den Teilchen physikalisch haftet. Diese gelösten Feinstoffe bilden einen Matsch, der ähnlich wie Öl schmiert.

Die Substratmischung, die zumindest 50 Gew.% Tiermist enthält, wird zunächst mit der Waage 1 gewogen, dann auf der Siloplatte 2 abgelagert. Hier kann das Substrat durch Inaugenscheinnahme geprüft werden, ob es geeignet ist. Gegebenenfalls wird ein anderes Substrat zugemischt, um beispielsweise den Anteil an nicht vergärbaren Stoffen, wie zum Beispiel Sand oder andere Verunreinigungen, herabzusetzen.

Falls das Substrat einen hohen Anteil langer Fasern enthält, kann es mit dem Schredder gehäckselt werden, wobei hier jedoch zu berücksichtigen ist, dass es für das vorliegende Verfahren vollkommen genügt, wenn die Fasern, insbesondere Stroh auf eine Länge von etwa 10 cm gehäckselt wird. Es ist nicht notwendig, alle Fasern weiter zu zerkleinern. Ein Häckseln der Faser auf eine Länge von etwa 10 cm lässt sich mit geringem Energiebedarf erreichen. Zum Häckseln der Fasern auf eine kürzere Länge würde ein Vielfaches der Energie benötigt werden. Für das vorliegende Verfahren genügen Schnittlängen von 5 cm bis 20 cm für eine ausreichende Homogenisierung der Substratmischung.

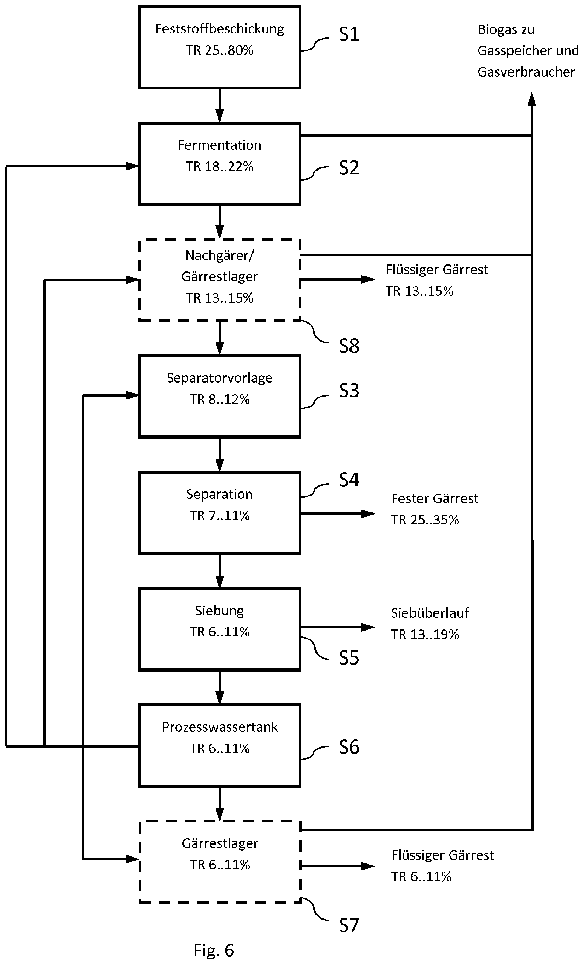

Die Substratmischung wird dann mittels der Feststoffbeschickung 6 und einer der beiden Fördereinrichtungen 29 zu einem der beiden Fermenter 4, 5 gefördert (Fig. 6 Schritt S1; Fig. 7). Die den Fermentern 4, 5 zugeführten Substrate weisen üblicherweise einen TR von 25 % bis 80 % auf.

Den Fermentern 4, 5 wird von der Pumpeinheit 24 Prozesswasser zugeführt, um den Anteil an TR beim Vergären im Fermenter (Schritt S2) auf zumindest 16 % einzustellen. Der TR im Fermenter wird, wie in Figur 6 dargestellt, typischerweise auf einen Bereich von 18 % bis 22 % eingestellt.

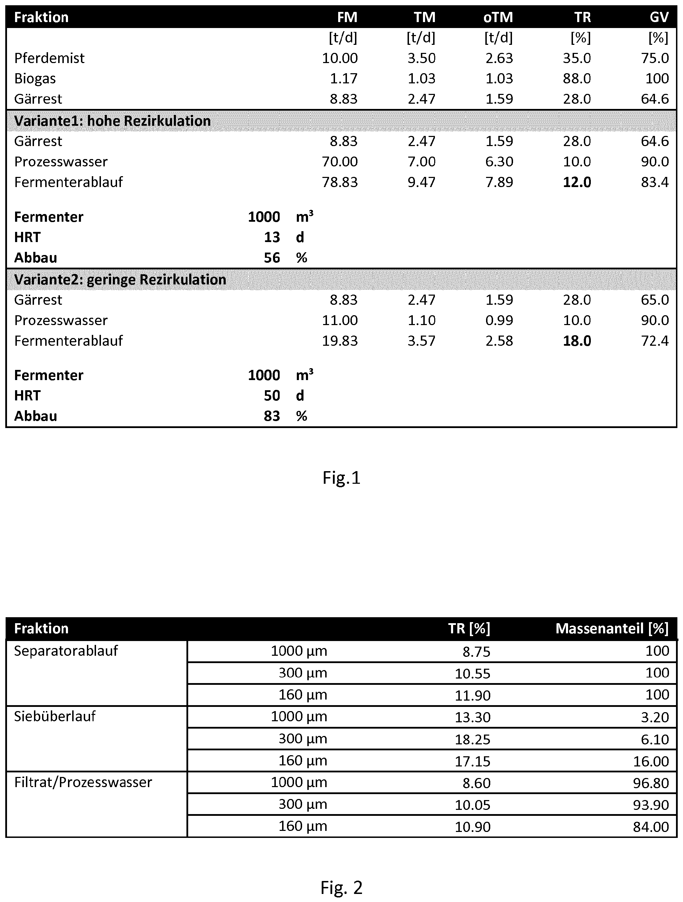

Figur 1 zeigt eine Tabelle mit der Massenbilanz eines Biogasreaktors zur Vergärung einer vor allem Pferdemist enthaltenden Substratmischung mit unterschiedlichem TR. Hierbei bedeuten die in der Tabelle angegebenen Abkürzungen folgendes:

FM     Frischmasse [t/d]
TM     Trockenmasse [t/d]
oTM    organische Trockenmasse [t/d]
TR     Trockenrückstand [%]

GV      Glühverlust [%]

Die Frischmasse ist die Masse der über die Fördereinrichtung 29 den Fermentern 4, 5 zugeführten Substratmischung und die Masse des den Fermentern 4, 5 zugeführten Prozesswassers. Die Substratmischung besteht bei den Beispielen in der Tabelle aus Figur 1 fast ausschließlich aus Pferdemist. Bei den in der Tabelle angegebenen Beispiel werden 10 Tonnen Substratmischung pro Tag dem Fermenter zugeführt.

Die Trockenmasse ist der Anteil der Stoffe der Substratmischung, die nicht Wasser sind. Dies sind vor allem organische Trockenmasse, Salze und zu einem geringen Anteil Sand. Der Anteil der Trockenmasse beträgt 3,5 Tonnen, die pro Tag dem Fermenter zugeführt werden. Hiervon entfallen 2,63 Tonnen auf die organische Trockenmasse.

Der Trockenrückstand TR ist der Rückstand, der sich beim Eindampfen der Substratmischung bei 105 °C ergibt. Er beträgt beim vorliegenden Ausführungsbeispiel 35 Gew.% und entspricht somit der Trockenmasse von 3,5 Tonnen an der gesamten zugeführten Substratmischung von 10 Tonnen pro Tag.

Der Glühverlust GV ist der organische Anteil des Trockenrückstandes, der bei 550 °C verglüht. Er wird auf die Trockenmasse bezogen und beträgt hier 75 Gew.%. Das Produkt aus Trockenrückstand und Glühverlust ist die organische Trockenmasse. Der Rest der Trockenmasse ist der Glührückstand, also anorganische Verbindungen wie Sand und Salze.

Aus den 10 Tonnen Substratmischung pro Tag entstehen 1,17 Tonnen Biogas pro Tag. Der tägliche Gärrest beträgt somit 8,83 Tonnen. Aus der organischen Trockenmasse entstehen 1,03 Tonnen Biogas pro Tag. Die Differenz von 0,14 Tonnen pro Tag zur Biogasproduktion bezüglich der Gesamtmasse der Substratmischung ergibt sich daraus, dass auch Wasser zur Biogasproduktion beiträgt. Der Wasseranteil, der bei der Biogasproduktion umgesetzt wird, beträgt 0,14 Tonnen pro Tag.

Das zugeführte Prozesswasser enthält einen TR von etwa 8 % bis 15 %. Dieser Anteil beruht darauf, dass in der Separationseinheit 33 nicht die gesamte Trockenmasse vom Prozesswasser getrennt werden kann. Werden dem Fermenter beispielsweise 70 Tonnen Prozesswasser pro Tag zugeführt (Fig.1, Variante1), dann bedeutet dies, dass dem Fermenter mittels des Prozesswassers 7 Tonnen Trockenmasse zugeführt werden. Diese 7 Tonnen Trockenmasse pro Tag umfassen 6,3 Tonnen organische Trockenmasse. Der Fermenterzulauf beträgt somit 10,5 Tonnen Trockenmasse (80 Tonnen Frischmasse) pro Tag; der Fermenterablauf enthält somit 9,47 Tonnen Trockenmasse pro Tag und die gesamte Frischmasse 78,8 Tonnen pro Tag. Die 9,47 Tonnen Trockenmasse pro Tag ergeben bezüglich der 78,8 Tonnen Frischmasse pro Tag einen Anteil von 12 %. Der TR und damit der TM-Gehalt im Fermenter beträgt somit 12 %, wenn 70 Tonnen Prozesswasser pro Tag zugeführt werden.

Bei einem Fermenter mit einem Volumen von 1000 $m^3$ bedeutet dies eine mittlere Verweildauer (HRT) der Substratmischung im Fermenter von 13 Tagen. Dies bedeutet eine Abbaurate von 56 %. Geht man bei einem alternativen Beispiel davon aus, dass wiederum 10 Tonnen vor allem Pferdemist enthaltende Substratmischung pro Tag dem Fermenter zugeführt werden, aber jedoch lediglich 11 Tonnen Prozesswasser pro Tag zugeführt werden (Fig.1, Variante2), dann ergibt sich als gesamter Anteil der zugeführten Trockenmasse pro Tag ein Wert von 4,6 Tonnen (21 Tonnen Frischmasse) und im Fermenterablauf ein Wert von 3,57 Tonnen Trockenmasse (19,8 Tonnen Frischmasse). Dies bedeutet einen TR von 18 %.

Da bei dem zweiten Beispiel wesentlich weniger Gesamtmasse pro Tag (21 Tonnen im Vergleich zu 80 Tonnen) zugeführt werden, ist auch das gesamte pro Tag zugeführte Volumen wesentlich geringer. Dies bedeutet auch eine wesentlich geringere Strömung durch den Fermenter. Die mittlere Verweildauer der Substratmischung im Fermenter verlängert sich dann auf etwa 50 Tage. Eine Verweildauer von 50 Tagen bedeutet eine Abbaurate von etwa 83 %.

Anhand dieser Tabelle kann man erkennen, dass je geringer die Zugabe von Prozesswasser zur Substratmischung ist, desto länger ist die Verweildauer und desto besser ist die Abbaurate. Daher besteht ein starkes Interesse, möglichst wenig Prozesswasser dem Fermenter zuzuführen und den Fermenter mit einem möglichst hohen TR zu betreiben.

Je größer der TR ist, desto zäher ist der Fermenterinhalt. Daher hat sich, wie es oben bereits erläutert ist, für landwirtschaftliche Substratmischungen für den Dauerbetrieb eine Obergrenze von ca. 8 % TR durchgesetzt. Es wurde jedoch überraschenderweise festgestellt, dass eine Substratmischung, welche überwiegend Tiermist enthält, auch bei einem relativ hohen TR eine ausreichend geringe Viskosität für den Dauerbetrieb in einem Fermenter aufweist. Daher wird beim erfindungsgemäßen Verfahren der Fermenter im Dauerbetrieb mit einem TR von zumindest 16 % betrieben. Es hat sich sogar gezeigt, dass im Einzelfall sogar Fermenter mit Fermenterinhalten mit noch höherem TR von zum Beispiel mindestens 18 % bzw. mindestens 19 % und sogar von mehr als 20 % wirtschaftlich sinnvoll betrieben werden können.

Die Erfinder führen diesen überraschenden Effekt darauf zurück, dass der Anteil an gelösten Feinstoffe in Tiermist wesentlich höher als bei nachwachsenden Rohstoffen für die Biogasproduktion ist und diese gelösten Feinstoffe die Viskosität des Fermenterinhaltes herabsetzen, selbst wenn die Substratmischung durch weitere die Viskosität nachteilig beeinflussender Bestandteile, wie zum Beispiel Stroh, verunreinigt ist.

In Figur 10 ist der Fließindex von Feinstoffe enthaltendem Prozesswasser unterschiedlicher Substratmischungen gegen den Konsistenzfaktor aufgetragen. Daraus kann gemäß dem Fließgesetz von Ostwald und deWaele die Viskosität des Prozesswassers unterschiedlicher Substratmischungen berechnet werden. Ein Fließindex von m=1 würde ein newton'sches Fluid beschreiben. Ein Fließindex von m<1 charakterisiert scherratenabhängig ein strukturviskoses Fluid.

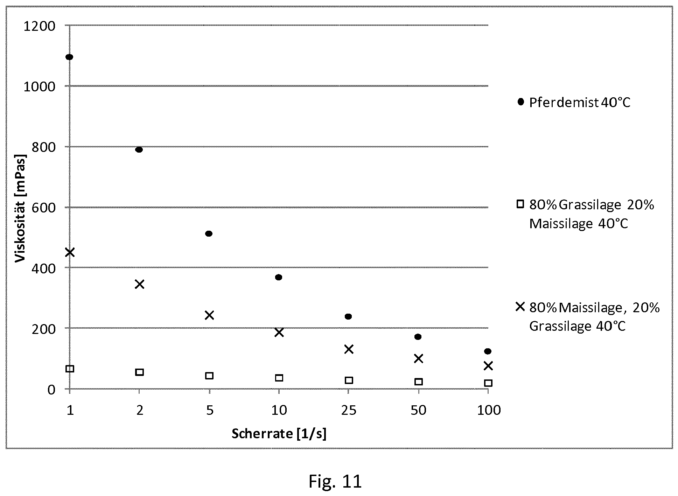

Bei der Vergärung von Tiermist ist wegen des hohen Anteils an Feinstoffen eine höhere Viskosität als bei der reinen Vergärung von nachwachsenden Rohstoffen im Prozesswasser zu messen. Figur 11 zeigt die Änderung der Viskosität eines Prozesswassers, welches Feinstoffe enthält, mit der Scherrate bei 40°C. Daher ist bei der Vergärung von Tiermist - bei gleichem TR im Fermenter wie bei einer Vergärung nachwachsender Rohstoffe - ein höherer Anteil des TR in dem Prozesswasser zu finden. Dieses Prozesswasser wurde mit einer Siebung des Fermenterinhalts mit einer Plansiebmaschine mit einer Maschenweite von 500 $\mu$m gewonnen und entspricht der Flüssigphase des Fermenterinhaltes, weshalb diese Viskosität auch für die Flüssigphase charakteristisch ist.

Bei der Vergärung von Tiermist ist Wasser physikalisch an Feinstoffe gebunden, sodass sich ein "schmierender" Matsch bildet. Durch dieses "Schmieren" ist die Viskosität des Fermenterinhalts so gering, so dass eine Biogasanlage mit einem TR von zumindest 16% auf Dauer wirtschaftlich betrieben werden kann und der energetische Aufwand zum Rühren des Fermenterinhalts gering ist.

Durch das Betreiben des Fermenters mit einem TR von mindestens 16 % kann die Abbaurate im Vergleich zu herkömmlichen Verfahren und damit die Effizienz der Biogasproduktion wesentlich gesteigert werden.

Das in den Fermentern 4, 5 abgebaute Gärsubstrat wird zunächst dem Vorlagehomogenisierungstank 15 zugeführt, indem es mit zurückgeführtem Prozesswasser auf einen TR von etwa 8 % bis 12 % verdünnt wird (Fig. 6 Schritt S3). Vom Vorlagehomogenisierungstank 15 wird das abgebaute, verdünnte Gärsubstrat dem Pressschneckenseparator 17 zugeführt, in der es mechanisch entwässert wird bzw. in einen festen Gärrest mit einem TR von 25 % bis 35 % und in Prozesswasser getrennt wird (Fig. 6 Schritt S4). Das Prozesswasser weist einen TR von 7 % bis 11 % und insbesondere von 8 % bis 11 % auf.

Das abgetrennte Prozesswasser wird mittels eines Schwingsiebes 20 gesiebt, wobei ein musartiger Siebüberlauf mit einem TR von etwa 13 % bis 19 % produziert wird (Fig. 6 Schritt S5). Nach längerem Betrieb des Siebes kann der TR im Siebüberlauf bis auf etwa 22 % ansteigen. Die Tabelle in Figur 2 zeigt Siebungen mit Spaltenweiten von 1000 $\mu$m, 300 $\mu$m und 160 $\mu$m und die entsprechenden Trockenrückstände für den Siebüberlauf und das Filtrat bzw. die entsprechenden Massenanteile. Wie man anhand der Tabelle aus Figur 2 erkennen kann, ist die Wahl der Spaltenweite des Spaltensiebes des Pressschneckenseparators 17 ohne großen Einfluss auf die Entwässerungsleistung des Pressschneckenseparators 17. Die Spaltenweite kann im Bereich von 250 $\mu$m bis 1000 $\mu$m liegen. Spaltensiebe mit größerer Spaltenweite sind kostengünstiger und stabiler, weshalb sie bevorzugt werden.

Der Siebüberlauf wird über die Fördereinrichtung 21 und die Fördereinrichtung 18 zum Auffangbunker 35 für feste Gärreste gefördert.

Das durch die Siebung weiter gereinigte Prozesswasser wird dem Prozesswassertank 23 zugeführt.

Aus dem Prozesswassertank 23 wird mittels der Pumpeneinheit 24 Prozesswasser den Fermentern 4 und 5 und überschüssiges Prozesswasser dem Gärrestlager 25 zugeführt (Fig. 6 Schritt S6).

Im Gärrestlager wird der flüssige Gärrest mit einem TR von etwa 6 % bis 11 % gespeichert (Fig. 6 Schritt S7).

In Figur 6 ist auch eine alternative Ausführungsform dargestellt, bei welcher die Fermenter direkt mit dem Gärrestlager 25 verbunden sind, wobei die Gärsubstrate im Gärrestlager 25 zwischengespeichert werden (Fig. 6 Schritt S8), bevor sie dem Vorlagehomogenisierungstank 15 zugeführt werden. Hier kann das Gärsubstrat nachgären. Bei diesem Verfahren stellt sich ein TR im Gärrestlager von etwa 13 % bis 15 % ein.

Die wesentliche Trennung der abgebauten Gärreste in festen Gärrest und Prozesswasser findet in der ersten Separationsstufe statt (Schritt S4). Für den grundsätzlichen Betrieb der Biogasanlage ist eine zusätzliche Separationsstufe nicht notwendig. Die Erfinder haben jedoch festgestellt, dass es bei der Verarbeitung von Tiermist enthaltenden Substratmischungen erhebliche Probleme bei Pressschneckenseparatoren 17 gibt, die vor allem durch Sand und auch durch feines Fasermaterial im Gärsubstrat verursacht werden. Aufgrund der Rückführung des Prozesswassers in die Fermenter werden diese Störstoffe im Kreislauf angereichert.

Diese Störstoffe führen zu einer Verstopfung der Siebspalten, so dass die Siebe nicht über ihre gesamte Länge arbeiten. Im Pressschneckenseparator 17 entstehen hierdurch Pfropfen, die sich hin und wieder schlagartig lösen und so zu einem Durchbruch von nicht getrenntem Material führen.

Weiterhin verursacht der Sand einen hohen Verschleiß an Schnecke und Sieb. Ohne die Siebeinheit müsste der Pressschneckenseparator 17 täglich gereinigt werden.

Durch die der ersten Separationsstufe nachgeschalteten zweiten Separationsstufe 20 in Form eines Schwingsiebs können diese Probleme vermieden oder auf ein verträgliches Maß reduziert werden, da durch das Schwingsieb 20 verhindert wird, dass sich Sand und faserartige Störstoffe nicht im Kreislauf anreichern. Das Vorsehen der Siebeinheit vereinfacht erheblich den Dauerbetrieb der Biogasanlage und macht sie wesentlich zuverlässiger für den Betrieb mit Tiermist enthaltenden Substratmischungen. Das Vorsehen der zweiten Separationsstufe in Form einer Siebeinheit stellt einen selbstständigen Erfindungsgedanken dar, wobei auch andere Separationseinrichtungen, die zum Trennen von Sand und faserartigen Störstoffen vom Prozesswasser geeignet sind, verwendet werden können.

Anstelle eines Pressschneckenseparators 17 kann auch ein Walzenseparator vorgesehen sein. Ein solcher Walzenseparator ist beispielsweise aus der DE 39 41 916 A1 bekannt. Ein Walzenseparator weist eine Siebwalze bzw. Siebtrommel auf, die für Flüssigkeiten durchlässig ist. Am Umfang der drehbar angetriebenen Siebwalze sind Abstreifelemente bzw. Druckwalzen angeordnet, so dass der Flüssigkeitsanteil (Filtrat) des Gärrestes durch die Siebwalze gedrückt wird und der feste Gärrest (Überstand) auf der Oberfläche der Siebwalze zurück bleibt und von dort abgestriffen wird.

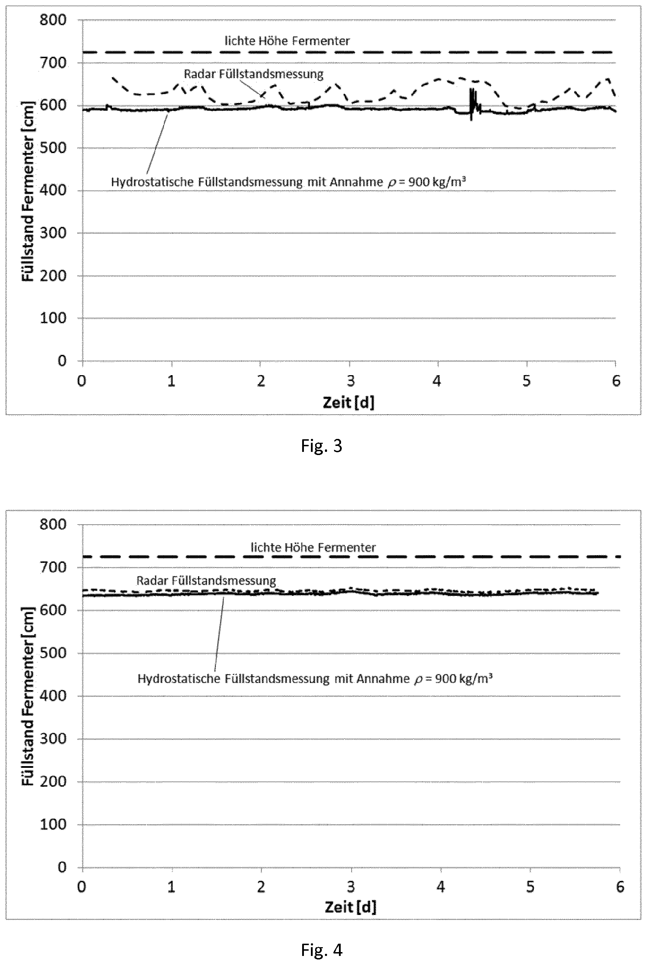

Bei vor allem Tiermist enthaltenden Substratmischungen kann ein hoher Anteil an Feinstoffen enthalten sein. Feinstoffe sind vor allem kleine Strohstaubpartikel. Durch die oben erläuterte Prozesswasserrückführung können die Feinstoffe im Fermenter angereichert werden, da sie weder in der ersten noch in der zweiten Separationsstufe vollständig vom Prozesswasser getrennt werden können. Wird eine Feinstoffe enthaltende Substratmischung vergärt, und der Fermenterinhalt mit einem Hauptrührwerk, dem Rührwerk 7, laminar umgewälzt, dann kann nicht das gesamte Biogas aus dem Fermenterinhalt entweichen. Es wird angenommen, dass kleine Bläschen aus Biogas aufgrund des hohen Anteils an Feinstoffen kombiniert mit der hohen Viskosität der Flüssigphase im Fermenter an den Feinstoffen haften bleiben und so nicht aus dem Fermenterinhalt austreten können. Es zeigt sich im Betrieb ein periodisches Akkumulieren von Mikrogasblasen im Fermenter. Dies hat zur Folge, dass der Fermenterfüllstand binnen weniger Stunden um ca. 10 % bis 20 % ansteigt. Die Periodendauer beträgt ca. 16 bis 20 Stunden. Misst man den Füllstand mit einer konventionellen hydrostatischen Füllstandssonde, werden diese periodischen Füllstandsänderungen nicht mitgemessen, da die Wassersäule über der Sonde bei der Akkumulation von Gasblasen im Fermenter konstant bleibt (Fig. 3). Mit einer Radar-Füllstandsmessung ist die Füllstandsänderungen erkennbar.

Beim erfindungsgemäßen Verfahren wird im Bereich benachbart zur oberen Oberfläche des Fermenterinhaltes dieser mit einem Nebenrührwerk, dem Stabmixer 10, turbulent umgewälzt. Hierdurch werden lokal starke Scherraten erzielt, die das Biogas von den Feinstoffen lösen und so das Biogas sicher und zuverlässig freisetzen. Der Füllstand bleibt dann auch bei einer Radar-Füllstandsmessung konstant (Fig. 4). Die Zugabe von teuren Anti-Schaummitteln oder Pflanzenöl wird vermieden und die Biogasanlage kann selbst mit einer Feinstoffe enthaltenden Substratmischung zuverlässig betrieben werden.

Vorzugsweise wird im Wesentlichen der gesamte Fermenterinhalt laminar umgewälzt und lediglich in einem kleinen Bereich wird die laminare Strömung von der turbulenten Strömung überlagert.

Das Hauptrührwerk kann mit einer Reynoldszahl von nicht mehr als 50 und das Nebenrührwerk mit einer Reynoldszahl von zumindest 100 betrieben werden. Eine Reynoldszahl im Bereich von 5 bis 20 für das Hauptrührwerk 7 bewirkt eine vollständige und effiziente Durchmischung eines dickflüssigen Fermenterinhalts mit wenig Energieaufwand. Der spezifische Energieeintrag ist beim Stabmixer 10 wesentlich größer als beim langsam drehenden Paddelrührwerk 7. Jedoch wird vom Stabmixer wesentlich weniger Masse umgewälzt, so dass der Stabmixer die Gesamtenergiebilanz der Biogasanlage kaum beeinträchtigt. Das Nebenrührwerk wird vorzugsweise mit einer Reynoldszahl von zumindest 300 bzw. zumindest 400 betrieben. Es kann auch mit noch größeren Reynoldszahlen betrieben werden.

Beim obigen Ausführungsbeispiel wird ein Stabmixer bzw. Propellerrührwerk verwendet. Anstelle des Stabmixers 10 kann ein beliebiges anderes Rührwerk verwendet werden, mit welchem der Fermenterinhalt turbulent und lokal begrenzt umgewälzt werden kann.

Die Biogasanlage weist eine zentrale Steuereinrichtung auf, die mit allen einstellbaren und den Betrieb der Biogasanlage beeinflussenden Elementen, wie z.B. Pumpen, Ventile, Siebe, Heizungen, Rührwerke, Sensoren, etc. verbunden ist, so dass die Steuereinrichtung selbsttätig das oben erläuterten Verfahren ausführen kann.

**Bezugszeichenliste**

| 1 | Waage | 20 | Schwingsieb |
|---|---|---|---|
| 2 | Siloplatte | 21 | Fördereinrichtung |
| 3 | Schredder | 22 | Leitung |
| 4 | erster Fermenter | 23 | Prozesswassertank |
| 5 | zweiter Fermenter | 24 | Pumpeinheit |
| 6 | Feststoffbeschickung | 25 | Gärrestlager |
| 7 | Paddelrührwerk | 26 | Rezirkulatleitung |
| 8 | Rührwelle | 27 | Gasleitung |
| 9 | Paddel | 28 | Gasspeicher |
| 10 | Stabmixer | 29 | Fördereinrichtung |
| 11 | Welle | 30 | Auslassöffnung |
| 12 | Propeller | 31 | Blockheizkraftwerk |
| 13 | Austragsöffnung | 32 | Notfackel |

(fortgesetzt)

| 14 | Leitung | 33 | Separationseinheit |
|----|---------|----|--------------------|
| 15 | Vorlagehomogenisierungstank | 34 | Sandabscheider |
| 16 | Pumpe | 35 | Auffangbunker |
| 17 | Pressschneckenseparator | 36 | Rezirkulatleitung |
| 18 | Fördereinrichtung | | |
| 19 | Leitung | | |

**Patentansprüche**

1. Verfahren zur Herstellung von Biogas durch kontinuierliche einstufige Vergärung von einer Substratmischung mit einem Biogasfermenter, einer Separationseinheit zum Trennen des im Biogasfermenter vergorenen Gärsubstrates in Gärrest und Prozesswasser, wobei Prozesswasser in den Biogasfermenter zurück geführt wird,
   **dadurch gekennzeichnet,**
   **dass** die Substratmischung zumindest 50 Gew.% Tiermist enthält und
   **dass** die Menge an zurück geführtem Prozesswasser derart eingestellt wird, dass im Biogasfermenter der Fermenterinhalt einen Trockenrückstand (TR) von zumindest 16% aufweist.

2. Verfahren nach Anspruch 1,
   **dadurch gekennzeichnet,**
   **dass** der Fermenterinhalt im Biogasfermenter einen Trockenrückstand von mindestens 17%, bevorzugt mindestens 18% und besonders bevorzugt mindestens 20% aufweist.

3. Verfahren nach Anspruch 1-2,
   **dadurch gekennzeichnet,**
   **dass** die Substratmischung einen Anteil von zumindest 80 Gew.% Tiermist enthält.

4. Verfahren nach Anspruch 1-3,
   **dadurch gekennzeichnet,**
   **dass** die Flüssigphase des Fermenterinhalts bei einer Temperatur von 40°C und einer Scherrate von 10 s$^{-1}$ eine Viskosität von mindestens 200 mPas oder mindestens 300 mPas, insbesondere mindestens 400 mPas oder mindestens 500 mPas aufweist.

5. Verfahren nach Anspruch 1-4,
   **dadurch gekennzeichnet,**
   **dass** die Flüssigphase einen Trockenrückstand von mindestens 7%, von mindestens 9%, oder von mindestens 11% aufweist.

6. Verfahren nach Anspruch 1-5,
   **dadurch gekennzeichnet,**
   **dass** der Fermenterinhalt einen TR-Gehalt von zumindest 16% aufweist und mit einem Hauptrührwerk laminar umgewälzt wird, und im Bereich benachbart zur oberen Oberfläche des Fermenterinhaltes dieser mit einem Nebenrührwerk turbulent umgewälzt wird.

7. Verfahren nach Anspruch 6,
   **dadurch gekennzeichnet,**
   **dass** zum laminaren Umwälzen ein Rührwerk mit vertikal angeordneter Rührwelle verwendet wird, so dass der Fermenterinhalt laminar, kreisförmig und etwa horizontal umgewälzt wird.

8. Verfahren nach Anspruch 6-7,
   **dadurch gekennzeichnet,**
   **dass** das Hauptrührwerk mit einer Reynoldszahl von nicht mehr als 50 und das Nebenrührwerk mit einer Reynoldszahl von zumindest 100 betrieben wird.

9. Biogasanlage zur kontinuierlichen Vergärung von zumindest 50 Gew.% Tiermist enthaltenden Substratmischungen mit einem einstufigen Biogasfermenter (4, 5), einer Separationseinheit (33) zum Trennen des im Biogasfermenter (4, 5) vergorenen Gärsubstrates in Gärrest und Prozesswasser, wobei eine Rezirkulatleitung (36) vorgesehen ist, um Prozesswasser in den Biogasfermenter (4, 5) zurückzuführen und eine Strömungseinrichtung vorgesehen ist, um den Strom an zurückgeführten Prozesswasser derart einzustellen, dass im Fermenter (4, 5) ein Trockenrückstand von zumindest 16 % vorliegt.

10. Biogasanlage nach Anspruch 9,
**dadurch gekennzeichnet,**
**dass** die Stömungseinrichtung eine Pumpe und/oder zumindest ein Drosselventil aufweist.

11. Biogasanlage nach Anspruch 9 oder 10,
**dadurch gekennzeichnet,**
**dass** die Separationseinheit einstufig ausgebildet ist und als Dekanter ausgeführt ist.

12. Biogasanlage nach Anspruch 9 oder 10,
**dadurch gekennzeichnet,**
**dass** die Separationseinheit zweistufig ausgebildet ist, wobei eine erste Separationsstufe als Separator mit Pressschnecke oder als Walzenseparator und die zweite Separationsstufe als Schwingsieb ausgeführt ist, wobei die zweite Separationsstufe eine kleinere Maschenweite als die erste Separationsstufe aufweist.

13. Biogasanlage, nach einem der Ansprüche 9 bis 12,
zur kontinuierlichen Vergärung von zumindest 50 Gew.% Tiermist enthaltenden Substratmischungen mit einem einstufigen Biogasfermenter (4, 5), einer Separationseinheit (33) zum Trennen des im Biogasfermenter (4, 5) vergorenen Gärsubstrates in Gärrest und Prozesswasser,
**dadurch gekennzeichnet,**
**dass** die Separationseinheit (33) einen Vorlagehomogenisierungstank (15) aufweist, wobei von einem Prozesswassertank (23) eine Rezirkulatleitung (26) zum Vorlagehomogenisierungstank führt, um Prozesswasser zum Einstellen des Trockenrückstandes vor dem Separieren in die Separationseinheit (33) zurückzuführen.

14. Biogasanlage zur kontinuierlichen Vergärung von Tiermist mit einem Biogasfer-I menter, nach einem der Ansprüche 9 bis 13, mit
einem Hauptrührwerk zum laminaren Umwälzen,
**dadurch gekennzeichnet,**
**dass** im Bereich benachbart zur oberen Oberfläche des Fermenterinhaltes ein Nebenrührwerk zum turbulenten Umwälzen vorgesehen ist.

15. Biogasanlage nach einem der Ansprüche 9 bis 14,
**dadurch gekennzeichnet,**
**dass** eine Steuereinrichtung zum Ansteuern der Biogasanlage vorgesehen ist, die zum Durchführen eines Verfahrens nach einem der Ansprüche 1 bis 8 ausgebildet ist.

**Claims**

1. Method for preparing biogas by continuous single-stage fermentation of a substrate mixture using a biogas fermenter, a separation unit for the separation of fermentation residue and process water from the fermentation substrate fermented in the biogas fermenter, wherein the process water is fed back into the biogas fermenter,
**characterised in**
**that** the substrate mixture contains at least 50% animal dung and
**that** the amount of recycled process water is set so that the fermenter content in the biogas fermenter has a dry residue (TR) content of at least 16%.

2. Method according to claim 1,
**characterised in that**
the fermenter content in the biogas fermenter has a dry residue (TR) of at least 17 %, preferably at least 18 % and especially preferably at least 20 %.

3. Method according to claims 1-2,

**characterised in that**
the substrate mixture has an animal dung content of at least 80% by weight.

4. Method according to claims 1-3,
**characterised in that**
the liquid phase of the fermenter content at a temperature of 40°C and a shear rate of 10 s$^{-1}$ has a viscosity of at least 200 mPas or at least 300 mPas, in particular at least 400 mPas or at least 500 mPas.

5. Method according to claims 1-4,
**characterised in that**
the liquid phase has a dry residue of at least 7%, of at least 9%, or of at least 11%.

6. Method according to claims 1-5,
**characterised in that** the fermenter content has a dry residue (TR) content of at least 16% and is circulated with laminar flow by a main agitator and in the area adjacent to the upper surface of the fermenter content the latter is circulated with turbulent flow by a secondary agitator.

7. Method according to claim 6,
**characterised in that**
for the laminar flow circulation an agitator with a vertically mounted agitator shaft is used, so that the fermenter content is circulated with laminar flow, circularly and roughly horizontally.

8. Method according to claims 6-7,
**characterised in that**
the main agitator is operated with a Reynolds number of no more than 50 and the secondary agitator with a Reynolds number of at least 100.

9. Biogas plant for the continuous fermentation of substrate mixtures containing at least 50% animal dung with a single-stage biogas fermenter (4, 5), a separation unit (33) for the separation of fermentation residue and process water from the fermentation substrate fermented in the biogas fermenter (4, 5), wherein a recirculation pipe (36) is provided for recycling process water into the biogas fermenter (4, 5), and a flow device is provided to adjust the flow of recycled process water so that there is a dry residue of at least 16% in the fermenter (4, 5).

10. Biogas plant according to claim 9,
**characterised in that**
the flow device has a pump and/or at least a restrictor valve.

11. Biogas plant according to claim 9 or 10,
**characterised in that**
the separation unit is a single-stage unit, in particular in the form of a decanter.

12. Biogas plant according to claim 9 or 10,
**characterised in that**
the separation unit is two-stage in form, wherein preferably a first separation stage is designed as a separator with pressing screw or as a roller separator, and the second separation stage is in particular in the form of a vibrating screen, wherein preferably the second separation stage has a smaller screen aperture than the first separation stage.

13. Biogas plant, in particular according to any of claims 9 to 12,
for the continuous fermentation of substrate mixtures containing at least 50% animal dung with a single-stage biogas fermenter (4, 5), a separation unit (33) for the separation fermentation residue and process water from the fermentation substrate fermented in the biogas fermenter (4, 5),
**characterised in that**
the separation unit (33) has a feedstock homogenisation tank (15), wherein a recirculation pipe (26) leads from a process water tank (23) to the feedstock homogenisation tank, in order to recycle process water for adjusting the dry residue before separation in the separation unit (33).

14. Biogas plant for the continuous fermentation of animal dung using a biogas fermenter, in particular according to any of claims 9 to 13, with a main agitator for circulation with laminar flow,

**characterised in that**
a secondary agitator for circulation with turbulent flow is provided in the area adjacent to the upper surface of a fermenter content.

15. Biogas plant according to any of claims 9 to 14,
**characterised in that** a control unit is provided to drive the biogas plant, which is made to execute a method according to one of claims 1 to 8.

**Revendications**

1. Procédé pour la fabrication de biogaz par fermentation continue à un seul étage d'un mélange substrat avec un fermenteur de biogaz, une unité de séparation pour la séparation du reste de fermentation et de l'eau de traitement du substrat fermenté dans le fermenteur de biogaz, de sorte que l'eau de traitement est ramenée dans le fermenteur de biogaz,
**caractérisé en ce**
**que** le mélange substrat contient au moins 50 % de fumier animal et
**que** la quantité d'eau de traitement ramenée est réglée de telle façon que le contenu de fermentation dans le fermenteur de biogaz présente un résidu sec (TR) d'au moins 16 %.

2. Procédé selon la revendication 1,
**caractérisé en ce que**
le contenu de fermentation dans le fermenteur de biogaz présente un résidu sec d'au moins 17 %, de préférence d'au moins 18 %, et de manière particulièrement préférée d'au moins 20 %.

3. Procédé selon la revendication 1 à 2,
**caractérisé en ce que**
le mélange substrat contient une part d'au moins 80 % en poids de fumier animal.

4. Procédé selon la revendication 1 à 3,
**caractérisé en ce que**
la phase liquide du contenu du fermenteur à une température de 40° C et un taux de cisaillement de $10 \text{ s}^{-1}$ présente une viscosité d'au moins 200 mPa ou d'au moins 300 mPa, en particulier d'au moins 400 mPa ou d'au moins 500 mPa.

5. Procédé selon la revendication 1 à 4,
**caractérisé en ce que** la phase liquide présente un résidu sec d'au moins 7 %, d'au moins 9 % ou d'au moins 11 %.

6. Procédé selon la revendication 1 à 5,
**caractérisé en ce que**
le contenu du fermenteur présente une teneur de résidu sec (TR) d'au moins 16 % et est brassé de façon laminaire avec un agitateur principal, et dans la région au voisinage de la surface supérieure du contenu du fermenteur, celui-ci est brassé de manière turbulente avec un agitateur auxiliaire.

7. Procédé selon la revendication 6,
**caractérisé en ce que**
pour le brassage laminaire on utilise un agitateur avec un arbre d'agitateur agencé verticalement, de sorte que le contenu du fermenteur est brassé de manière laminaire, sous forme circulaire et environ horizontalement.

8. Procédé selon la revendication 6 à 7,
**caractérisé en ce que**
l'agitateur principal est amené à fonctionner avec un nombre de Reynolds qui ne dépasse pas 50 et l'agitateur auxiliaire est amené à fonctionner avec un nombre de Reynolds d'au moins 100.

9. Installation de biogaz pour la fermentation continue de mélanges substrat contenant au moins 50 % de fumier animal avec un fermenteur de biogaz (4, 5) à un seul étage, une unité de séparation (33) pour la séparation du reste de fermentation et de l'eau de traitement du substrat fermenté dans le fermenteur de biogaz (4, 5), un conduit de recirculation (36) étant prévu afin de ramener de l'eau de traitement dans le fermenteur de biogaz (4, 5), et un système d'écoulement étant prévu afin de régler l'écoulement d'eau de traitement ramené de telle façon qu'un résidu sec d'au moins 16 % se présente dans le fermenteur (4, 5).

10. Installation de biogaz selon la revendication 9,
**caractérisée en ce que**
le système d'écoulement comprend une pompe et/ou au moins une vanne à étranglement.

11. Installation de biogaz selon la revendication 9 ou 10,
**caractérisée en ce que**
l'unité de séparation est réalisée à un seul étage, et est réalisée sous forme de décanteur.

12. Installation de biogaz selon la revendication 9 ou 10,
**caractérisée en ce que**
l'unité de séparation est réalisée à deux étages, un premier étage de séparation étant réalisé sous forme de séparateur avec une vis-presse ou sous forme de séparateur à cylindre, et le second étage de séparation étant réalisé sous forme de crible oscillant, le second étage de séparation présentant une largeur de mailles plus petite que le premier étage de séparation.

13. Installation de biogaz selon l'une des revendications 9 à 12,
pour la fermentation continue de mélanges substrat contenant au moins 50% de fumier animal avec un fermenteur de biogaz à un seul étage (4, 5), une unité de séparation (33) pour la séparation du reste de fermentation et de l'eau de traitement du substrat fermenté dans le fermenteur de biogaz (4, 5),
**caractérisée en ce que**
l'unité de séparation (33) comprend un réservoir d'homogénéisation préliminaire (15), un conduit de recirculation (26) menant depuis un réservoir d'eau de traitement (23) jusqu'au réservoir d'homogénéisation préliminaire, afin de ramener de l'eau de traitement dans l'unité de séparation (33) pour régler le résidu sec avant la séparation.

14. Installation de biogaz pour la fermentation continue de fumier animal avec un fermenteur de biogaz selon l'une des revendications 9 à 13, comprenant un agitateur principal pour un brassage laminaire,
**caractérisée en ce que**
dans la région au voisinage de la surface supérieure du contenu d'un fermenteur il est prévu un agitateur auxiliaire pour le brassage turbulent.

15. Installation de biogaz selon l'une des revendications 9 à 14,
**caractérisée en ce**
**qu'**il est prévu un système de commande pour commander l'installation de biogaz, lequel est fait pour exécuter un procédé selon l'une des revendications 1 à 8.

| Fraktion | FM | TM | oTM | TR | GV |
|---|---|---|---|---|---|
| | [t/d] | [t/d] | [t/d] | [%] | [%] |
| Pferdemist | 10.00 | 3.50 | 2.63 | 35.0 | 75.0 |
| Biogas | 1.17 | 1.03 | 1.03 | 88.0 | 100 |
| Gärrest | 8.83 | 2.47 | 1.59 | 28.0 | 64.6 |
| **Variante1: hohe Rezirkulation** | | | | | |
| Gärrest | 8.83 | 2.47 | 1.59 | 28.0 | 64.6 |
| Prozesswasser | 70.00 | 7.00 | 6.30 | 10.0 | 90.0 |
| Fermenterablauf | 78.83 | 9.47 | 7.89 | **12.0** | 83.4 |
| | | | | | |
| **Fermenter** 1000 m³ | | | | | |
| **HRT** 13 d | | | | | |
| **Abbau** 56 % | | | | | |
| **Variante2: geringe Rezirkulation** | | | | | |
| Gärrest | 8.83 | 2.47 | 1.59 | 28.0 | 65.0 |
| Prozesswasser | 11.00 | 1.10 | 0.99 | 10.0 | 90.0 |
| Fermenterablauf | 19.83 | 3.57 | 2.58 | **18.0** | 72.4 |
| | | | | | |
| **Fermenter** 1000 m³ | | | | | |
| **HRT** 50 d | | | | | |
| **Abbau** 83 % | | | | | |

Fig.1

| Fraktion | | TR [%] | Massenanteil [%] |
|---|---|---|---|
| Separatorablauf | 1000 µm | 8.75 | 100 |
| | 300 µm | 10.55 | 100 |
| | 160 µm | 11.90 | 100 |
| Siebüberlauf | 1000 µm | 13.30 | 3.20 |
| | 300 µm | 18.25 | 6.10 |
| | 160 µm | 17.15 | 16.00 |
| Filtrat/Prozesswasser | 1000 µm | 8.60 | 96.80 |
| | 300 µm | 10.05 | 93.90 |
| | 160 µm | 10.90 | 84.00 |

Fig. 2

Fig. 3

Fig. 4

Fig. 5

Feststoffbeschickung
TR 25..80% — S1

Biogas zu
Gasspeicher und
Gasverbraucher

Fermentation
TR 18..22% — S2

Nachgärer/
Gärrestlager
TR 13..15%

Flüssiger Gärrest
TR 13..15%

S8

Separatorvorlage
TR 8..12% — S3

Separation
TR 7..11% — S4

Fester Gärrest
TR 25..35%

Siebung
TR 6..11%

Siebüberlauf
TR 13..19%

S5

Prozesswassertank
TR 6..11% — S6

Gärrestlager
TR 6..11%

Flüssiger Gärrest
TR 6..11%

S7

Fig. 6

Fig. 7

Fig. 8

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010019321 A1
- EP 1301583 B1
- DE 102010024639 A1
- DE 102004029854 A1
- DE 102008015609 A1
- DE 202008006843 U1
- DE 102005021503 A1
- DE 102007037187 A1
- DE 102009058588 A1
- DE 102010010091 A1
- DE 102010006609 A1
- WO 2010043424 A1
- EP 2064308 B1
- DE 4232449 B4
- DE 3941916 A1
- DE 202006003816 U1
- EP 2215241 B1
- DE 102005061039 A1
- DE 102006035794 A1
- EP 2535402 A1

**In der Beschreibung aufgeführte Nicht-Patentliteratur**

- **EUROHEAT ; POWER.** *Trennung von Hydrolyse und Methanisierung, Vom wertlosen Pferdemist zur wertvollen Energie,* 2011, vol. 40 (3), 38 ff
- **A. RUSHTON et al.** Solid-Liquid Filtration and Separation Technology. WILEY-VCH Verlag GmbH, 2000, 310